# EUROPÄISCHE PATENTSCHRIFT

(11) **EP 2 414 408 B1**
(45) Veröffentlichungstag und Bekanntmachung des Hinweises auf die Patenterteilung: **24.07.2013**
(21) Anmeldenummer: 10712011.5
(22) Anmeldetag: 27.03.2010
(51) Int. Cl.: C08F 20/34, C08F 20/60

(54) **KAMMFÖRMIGE, KATIONISCHE COPOLYMERE, DEREN HERSTELLUNG UND VERWENDUNG IN KOSMETISCHEN, PHARMAZEUTISCHEN UND DERMATOLOGISCHEN FORMULIERUNGEN**
CATIONIC COMB COPOLYMERS, PREPARATION THEREOF AND USE THEREOF IN COSMETIC, PHARMACEUTICAL AND DERMATOLOGICAL FORMULATIONS
COPOLYMÈRES CATIONIQUES EN PEIGNE, LEUR PRODUCTION ET LEUR UTILISATION DANS DES FORMULATIONS COSMÉTIQUES, PHARMACEUTIQUES ET DERMATOLOGIQUES

(30) Priorität: 01.04.2009 DE 102009015868
(43) Veröffentlichungstag der Anmeldung: 08.02.2012
(73) Patentinhaber: Clariant Finance (BVI) Limited, Road Town, Tortola (VG)
(72) Erfinder: KLUG, Peter, 63762 Großostheim (DE); FISCHER, Dirk, 55270 Klein-Winternheim (DE); LINDNER, Thomas, 68199 Mannheim (DE); KUNZE, Matthias, 60528 Frankfurt (DE); LOEFFLER, Matthias, 65510 Idstein (DE); MILDNER, Carina, 65931 Frankfurt am Main (DE); LO VASCO, Sebastiano, 63695 Glauburg (DE); CERNY, Tomas, 65795 Hattersheim (DE)
(74) Vertreter: Paczkowski, Marcus
(86) Internationale Anmeldenummer: PCT/EP2010/001952
(87) Internationale Veröffentlichungsnummer: WO 2010/112185

(56) Entgegenhaltungen:
- EP-A1- 1 464 658
- WO-A1-2006/062572
- WO-A1-2008/015136
- US-A- 3 861 948
- US-B1- 6 482 402

## Beschreibung

Die folgende Erfindung betrifft kammförmige, kationische Copolymere, ein Verfahren zur Herstellung solcher kammförmigen, kationischen Copolymere sowie die Verwendung dieser Copolymere in kosmetischen, pharmazeutischen und dermatologischen Formulierungen.

Verbraucherwünsche und Rheologie kosmetischer Produkte sind eng miteinander verknüpft. So wird. z. B. das visuelle Erscheinungsbild eines Shampoos oder einer Cremespülung und die Handhabbarkeit eines Öls durch die Viskosität beeinflusst. Die sensorischen Eigenschaften, wie Konsistenz oder Verteilbarkeit bestimmen das individuelle Profil eines Kosmetikproduktes. Nicht nur die Effektivität von Wirksubstanzen (z. B. Sonnenschutzfilter) sondern auch die Lagerstabilität der Formulierung steht in engem Zusammenhang mit den rheologischen Eigenschaften der Produkte.

Im kosmetischen Bereich kommt kationischen Polymeren als Conditioner eine tragende Rolle zu. Stand der Technik sind insbesondere Konditionierer auf Basis von Polydiallyldimethylammoniumchlorid und deren wasserlösliche Copolymere. Die Vielfalt der möglichen Strukturen und die damit verbundenen vielfältigen Anwendungsmöglichkeiten drücken sich nicht zuletzt in einer Vielzahl von Patenten aus, die seit Mitte der 70iger Jahre weltweit angemeldet wurden. Ein wesentlicher Nachteil der Konditionierer auf Basis von Polydiallyldimethylammoniumchlorid ist, dass diese in Formulierungen auf Basis von Ölen ein unzureichendes Verdickungsvermögen aufweisen. Zur Verbesserung der Konsistenz der Formulierungen werden häufig Fette und Wachse zugesetzt, die wiederum den Nachteil mit sich bringen, dass sie ein klebriges und stumpfes Hautgefühl bewirken.

Es bestand die Aufgabe, Substanzen für kosmetische, pharmazeutische und dermatologische Formulierungen zur Verfügung zu stellen, die sowohl konditionierende Eigenschaften für die Haut und für die Haare zeigen, und zugleich gute konsistenzgebende Eigenschaften auch in Formulierungen auf Basis von Ölen oder in Formulierungen mit hohem Ölanteil haben.

Es wurde überraschend gefunden, dass diese Aufgabe gelöst wird durch bestimmte kammförmige, kationische Copolymere, die Struktureinheiten enthalten, die sich formell ableiten von polymerisierbaren quaternären Ammoniumverbindungen und von polymerisierbaren nichtionischen Verbindungen, die Kohlenwasserstoffgruppen mit mindestens 12 Kohlenstoffatomen enthalten.

Gegenstand der Erfindung sind Copolymere enthaltend
a) 60,0 - 99,9 Gew.-% an einer oder mehreren Struktureinheiten hervorgegangen aus polymerisierbaren Substanzen der folgenden Strukturformel (I) 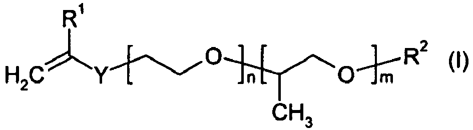 worin
   - R¹: Wasserstoff oder Methyl bedeutet,
   - Y: O, NR³, S, CH₂O, CH₂NR³, CH₂S, C(O), C(NR³), C(O)O oder C(O)NR³ bedeutet,
   - R²: einen linearen oder verzweigten gesättigten Alkylrest mit 12 bis 200 C-Atomen oder einen linearen oder verzweigten ein- oder mehrfach ungesättigten Alkenylrest mit 12 bis 200 C-Atomen bedeutet,
   - R³: Wasserstoff oder Methyl bedeutet und
   - n und m: jeweils unabhängig voneinander eine ganze Zahl von 0 bis 200 bedeuten,
b) 0,1 - 20,0 Gew.-% an einer oder mehreren Struktureinheiten hervorgegangen aus polymerisierbaren quaternären Ammoniumverbindungen, und
c) 0 - 20,0 Gew.% an einer oder mehreren nichtionischen Struktureinheiten hervorgegangen aus einer oder mehreren weiteren polymerisierbaren Substanzen.

Der oben in Komponente c) aufgeführte Begriff "hervorgegangen aus einer oder mehreren weiteren polymerisierbaren Substanzen" bedeutet im Rahmen der vorliegenden Erfindung, dass Komponente c) keine Verbindung umfasst, die eine Bedeutung der oben genannten Formel (I) annimmt.

Die erfindungsgemäßen Copolymere sind farblos bis opak und transparent, bewirken ein gutes Hautgefühl und verleihen den Haaren Glanz und eine gute Kämmbarkeit. Sie sind zur Herstellung von kosmetischen, pharmazeutischen und dermatologischen Formulierungen verschiedenster Art geeignet, und sind insbesondere mit Formulierungen auf wässrig-öliger Basis oder auf Öl-Basis gut kompatibel. Sie zeigen ein herausragendes Ölbindevermögen und können Öle gut verdicken. Darüber hinaus zeigen sie ein gutes Dispergiervermögen und sind zur stabilen Konsistenzgebung von Emulsionen und Cremes geeignet. Sie sind außerdem geeignet zur Formulierung von wasserfesten Sonnenschutzprodukten.

In WO 2004/041220 und WO 2004/041150 werden kosmetische Zubereitungen beschrieben, insbesondere Lippenstifte und Make-ups, enthaltend neben einer flüssigen Fettphase ein semikristallines Polymer aus einem C₁₄-C₂₄-α-Olefin und einem weiteren Monomer ausgewählt aus Carbonsäureestern, vorzugsweise C₁₄-C₂₄-Alkyl- oder C₁₁-C₁₅-Perfluoroalkyl-(meth)acrylaten, und N-Alkyl(meth)-acrylamiden.

In EP 1 681 046 werden kosmetische, pharmazeutische und dermatologische Zubereitungen beschrieben, die Copolymerwachse enthalten. Die Copolymerwachse enthalten Struktureinheiten, die formell abgeleitet sind von α-Olefinen mit 26 bis 60 C-Atomen, Derivaten von (Meth)acrylsäure wie Estern, Amiden oder Salzen und gegebenenfalls weiteren Monomeren.

In EP 1 693 047 werden kosmetische, pharmazeutische und dermatologische Zubereitungen beschrieben, die Copolymerwachse enthalten. Die Copolymerwachse enthalten Struktureinheiten, die abgeleitet sind von α-Olefinen mit 26 bis 60 C-Atomen, von Maleinsäureanhydrid, Maleinsäure oder deren Salzen und gegebenenfalls von weiteren Monomeren.

Bevorzugte erfindungsgemäße Copolymere enthalten 60,0 bis 99,9 Gew.-% an einer oder mehreren Struktureinheiten der Komponente a) hervorgegangen aus polymerisierbaren Substanzen der folgenden Strukturformel (I) 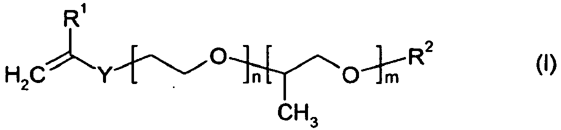 worin
- R¹: Wasserstoff oder Methyl bedeutet,
- Y: O, NR³, CH20, CH₂NR³, C(O)O oder C(O)NR³ und bevorzugt C(O)O oder C(O)NR³ bedeutet,
- R²: einen linearen oder verzweigten gesättigten Alkylrest mit 12 bis 200, bevorzugt mit 12 bis 58, besonders bevorzugt mit 12 bis 40 und insbesondere bevorzugt mit 12 bis 30 C-Atomen oder einen linearen oder verzweigten ein- oder mehrfach ungesättigten Alkenylrest mit 12 bis 200, bevorzugt mit 12 bis 58, besonders bevorzugt mit 12 bis 40 und insbesondere bevorzugt mit 12 bis 30 C-Atomen bedeutet,
- R³: Wasserstoff oder Methyl bedeutet und
- n und m: jeweils unabhängig voneinander eine ganze Zahl von 0 bis 200, bevorzugt von 0 bis 100, besonders bevorzugt von 0 bis 50 und insbesondere bevorzugt von 0 bis 30 bedeuten.

In den Verbindungen der Formel (I) ist Y insbesondere bevorzugt C(O)O.

Besonders bevorzugte erfindungsgemäße Copolymere enthalten 60,0 bis 99,9 Gew.% an einer oder mehreren Struktureinheiten der Komponente a), die hervorgegangen sind aus einer oder mehreren polymerisierbaren Verbindungen ausgewählt aus den Strukturformeln (Ia) und (Ib) 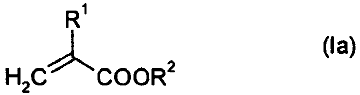 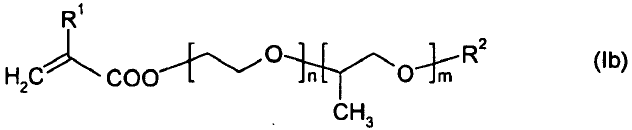 worin
- R¹: Wasserstoff oder Methyl bedeutet,
- R²: einen linearen oder verzweigten gesättigten Alkylrest mit 12 bis 200, bevorzugt mit 12 bis 58, besonders bevorzugt mit 12 bis 40 und insbesondere bevorzugt mit 12 bis 30 C-Atomen oder einen linearen oder verzweigten ein- oder mehrfach ungesättigten Alkenylrest mit 12 bis 200, bevorzugt mit 12 bis 58, besonders bevorzugt mit 12 bis 40 und insbesondere bevorzugt mit 12 bis 30 C-Atomen, bedeutet und
- n und m: jeweils unabhängig voneinander eine ganze Zahl von 0 bis 200, bevorzugt von 0 bis 100, besonders bevorzugt von 0 bis 50 und insbesondere bevorzugt von 0 bis 30 bedeuten und die Summe n + m eine Zahl von 1 bis 200, bevorzugt von 2 bis 100, besonders bevorzugt von 3 bis 50 und insbesondere bevorzugt von 3 bis 30, ist.

In einer bevorzugten Ausführungsform der Erfindung sind unter den Verbindungen der Formel (Ia) die (Meth)Acrylsäureester der Alkylalkohole bevorzugt, d. h. R² ist in diesen bevorzugten Verbindungen der Formel (Ia) ein Alkylrest. In einer besonders bevorzugten Ausführungsform der Erfindung ist R² in diesen Verbindungen ein linearer Alkylrest.

Die Bezeichnung "(Meth)Acryl" stellt im Rahmen der vorliegenden Erfindung eine abkürzende Bezeichnung für die beiden Bedeutungen "Acryl" und "Methacryl" dar.

In einer weiteren bevorzugten Ausführungsform der Erfindung sind unter den Verbindungen der Formel (Ia) die (Meth)acrylsäureester von Lauryl-, Myristyl-, Cetyl-, Stearyl-, Isostearyl-, Oleyl-, Octyldodecyl-, Behenyl- und Myricylalkohol bevorzugt.

In einer weiteren bevorzugten Ausführungsform der Erfindung sind unter den Verbindungen der Formel (Ia) die (Meth)acrylsäureester von Alkoholen mit 12 bis 22 C-Atomen bevorzugt. In dieser bevorzugten Ausführungsform der Erfindung sind unter den Verbindungen der Formel (Ia) die (Meth)acrylsäureester von Lauryl-, Myristyl-, Cetyl-, Stearyl-, Isostearyl-, Oleyl-, Octyldodecyl- und Behenylalkohol besonders bevorzugt und die (Meth)acrylsäureester von Lauryl-, Cetyl-, Stearyl-, Isostearyl-, Octyldodecyl- und Behenylalkohol insbesondere bevorzugt. Unter den soeben genannten (Meth)acrylsäureestern sind wiederum diejenigen der linearen Alkylalkohole bevorzugt.

In einer weiteren bevorzugten Ausführungsform der Erfindung sind unter den Verbindungen der Formel (Ib) die Substanzen ausgewählt aus (Meth)Acrylsäureestern von Alkyl-Polyethylenglykolen, von Alkyl-Polypropylenglykolen und von Alkyl-Polyethylen-polypropylenglykolen bevorzugt, d. h. R² ist in diesen bevorzugten Verbindungen der Formel (Ib) ein Alkylrest. In einer besonders bevorzugten Ausführungsform der Erfindung ist R² in diesen Verbindungen ein linearer Alkylrest.

In einer weiteren bevorzugten Ausführungsform der Erfindung ist "m" in den Verbindungen der Formel (Ib) 0, d. h. es handelt sich bei diesen Verbindungen um rein ethoxylierte Verbindungen. In dem Fall, dass m in den Verbindungen der Formel (Ib) 0 ist, bedeutet n eine ganze Zahl von 1 bis 200, bevorzugt von 2 bis 100, besonders bevorzugt von 3 bis 50 und insbesondere bevorzugt von 3 bis 30.

In einer weiteren bevorzugten Ausführungsform der Erfindung sind unter den Verbindungen der Formel (Ib) die Acryl- und/oder Methacrysäureester von Alkoholpolyglykolethern ausgewählt aus
a) Laurylethoxylaten, insbesondere

| | | |
|---|---|---|
| Genapol^{®} LA 020 | Laurylethoxylat mit 2 EO | C_{12/14}-Alkohol, linear |
| Genapol^{®} LA 030 | Laurylethoxylat mit 3 EO | C_{12/14}-Alkohol, linear |
| Genapol^{®} LA 040 | Laurylethoxylat mit 4 EO | C_{12/14}-Alkohol, linear |
| Genapol^{®} LA 050 | Laurylethoxylat mit 5 EO | C_{12/14}-Alkohol, linear |
| Genapol^{®} LA 070 | Laurylethoxylat mit 7 EO | C_{12/14}-Alkohol, linear |
| Genapol^{®} LA 080 | Laurylethoxylat mit 8 EO | C_{12/14}-Alkohol, linear |
| Genapol^{®} LA 090 | Laurylethoxylat mit 9 EO | C_{12/14}-Alkohol, linear |
| Genapol^{®} LA 110 | Laurylethoxylat mit 11 EO | C_{12/14}-Alkohol, linear |
| Genapol^{®} LA 200 | Laurylethoxylat mit 20 EO | C_{12/14}-Alkohol, linear |
| Genapol^{®} LA 2310 | Laurylethoxylat mit 23 EO | C_{12/14}-Alkohol, linear |
| Genapol^{®} LA 250 | Laurylethoxylat mit 25 EO | C_{12/14}-Alkohol, linear |

b) Cocosfettalkylethoxylaten, insbesondere

| | | |
|---|---|---|
| Genapol^{®} C 050 | C_{12/14/16}-Alkylethoxylat mit 5 EO | C_{12/14/16}-Alkohol, linear |
| Genapol^{®} C 100 | C_{12/14/16}-Alkylethoxylat mit 10 EO | C_{12/14/16}-Alkohol, linear |
| Genapol^{®} C 200 | C_{12/14/16}-Alkylethoxylat mit 20 EO | C_{12/14/16}-Alkohol, linear |
| Genapol^{®} C 250 | C_{12/14/16}-Alkylethoxylat mit 25 EO | C_{12/14/16}-Alkohol, linear |
| | | |
| Genapol^{®} U 100 | C_{14/16/18}-Alkylethoxylat mit 10 EO | C_{14/16/18}-Alkohol, ungesättigt |
| Genapol^{®} U 200 | C_{14/16/18}-Alkylethoxylat mit 20 EO | C_{14/16/18}-Alkohol, ungesättigt |
| | | |
| Genapol^{®} GS 080 | C₁₂₋₂₀-Alkylethoxylat mit 8 EO | C₁₂₋₂₀-Alkohol, linear |

c) Oleylethoxylaten, insbesondere

| | | |
|---|---|---|
| Genapol^{®} O 020 | Oleylethoxylat mit 2 EO | C_{16/18}-Alkohol, linear, ungesättigt |
| Genapol^{®} O 050 | Oleylethoxylat mit 5 EO | C_{16/18}-Alkohol, linear, ungesättigt |
| Genapol^{®} O 080 | Oleylethoxylat mit 8 EO | C₁₆₋₁₈-Alkohol, linear, ungesättigt |
| Genapol^{®} O 100 | Oleylethoxylat mit 10 EO | C_{16/18}-Alkohol, linear, ungesättigt |
| Genapol^{®} O 120 | Oleylethoxylat mit 12 EO | C_{16/18}-Alkohol, linear, ungesättigt |
| Genapol^{®} O 150 | Oleylethoxylat mit 15 EO | C_{16/18}-Alkohol, linear, ungesättigt |
| Genapol^{®} O 200 | Oleylethoxylat mit 20 EO | C_{16/18}-Alkohol, linear, ungesättigt |
| Genapol^{®} O 230 | Oleylethoxylat mit 23 EO | C_{16/18}-Alkohol, linear, ungesättigt |
| Genapol^{®} O 250 | Oleylethoxylat mit 25 EO | C_{16/18}-Alkohol, linear, ungesättigt |
| Genapol^{®} O 300 | Oleylethoxylat mit 30 EO | C_{16/18}-Alkohol, linear, ungesättigt |

d) Talkalkylethoxylaten, insbesondere

| | | |
|---|---|---|
| Genapol^{®} T 080 | Talkalkylethoxylat mit 8 EO | C_{16/18}-Alkohol, linear, ungesättigt |
| Genapol^{®} T 110 | Talkalkylethoxylat mit 11 EO | C_{16/18}-Alkohol, linear, ungesättigt |
| Genapol^{®} T 150 | Talkalkylethoxylat mit 15 EO | C_{16/18}-Alkohol, linear, ungesättigt |
| Genapol^{®} T 200 | Talkalkylethoxylat mit 20 EO | C_{16/18}-Alkohol, linear, ungesättigt |
| Genapol^{®} T 250 | Talkalkylethoxylat mit 25 EO | C_{16/18}-Alkohol, linear, ungesättigt |
| Genapol^{®} T 500 | Talkalkylethoxylat mit 50 EO | C_{16/18}-Alkohol, linear, ungesättigt |
| Genapol^{®} T 800 | Talkalkylethoxylat mit 80 EO | C_{16/18}-Alkohol, linear, ungesättigt |

e) Stearylalkoholethoxylaten, insbesondere

| | | |
|---|---|---|
| Genapol^{®} HS 020 | Stearylalkohol mit 2 EO | C_{16/18}-Alkohol, linear, gesättigt |
| Genapol^{®} HS 200 | Stearylalkohol mit 20 EO | C_{16/18}-Alkohol, linear, gesättigt |

g) Isotridecylethoxylaten, insbesondere

| | | |
|---|---|---|
| Genapol^{®} X 020 | Isotridecylethoxylat mit 2 EO | C₁₃-Alkohol, verzweigt |
| Genapol^{®} X 050 | Isotridecylethoxylat mit 5 EO | C₁₃-Alkohol, verzweigt |
| Genapol^{®} X 060 | Isotridecylethoxylat mit 6 EO | C₁₃-Alkohol, verzweigt |
| Genapol^{®} X 065 | Isotridecylethoxylat mit 6,5 EO | C₁₃-Alkohol, verzweigt |
| Genapol^{®} X 080 | Isotridecylethoxylat mit 8 EO | C₁₃-Alkohol, verzweigt |
| Genapol^{®} X 089 | Isotridecylethoxylat mit 8 EO | C₁₃-Alkohol, verzweigt |
| Genapol^{®} X 090 | Isotridecylethoxylat mit 9 EO | C₁₃-Alkohol, verzweigt |
| Genapol^{®} X 100 | Isotridecylethoxylat mit 10 EO | C₁₃-Alkohol, verzweigt |
| Genapol^{®} X 150 | Isotridecylethoxylat mit 15 EO | C₁₃-Alkohol, verzweigt |
| Genapol^{®} X 158 | Isotridecylethoxylat mit 15 EO | C₁₃-Alkohol, verzweigt |
| Genapol^{®} X 307 | Isotridecylethoxylat mit 30 EO | C₁₃-Alkohol, verzweigt |
| Genapol^{®} X 407 | Isotridecylethoxylat mit 40 EO | C₁₃-Alkohol, verzweigt |
| Genapol^{®} X 1003 | Isotridecylethoxylat mit 100 EO | C₁₃-Alkohol, verzweigt |
| Genapol^{®} X 1005 | Isotridecylethoxylat mit 100 EO | C₁₃-Alkohol, verzweigt |
| Genapol^{®} 1879 | Isotridecylethoxylat mit 15 EO | C₁₃-Alkohol, verzweigt |
| Genapol^{®} 3214 | Isotridecylethoxylat mit 25 EO | C₁₃-Alkohol, verzweigt |

i) Oxoalkoholethoxylaten, insbesondere

| | | |
|---|---|---|
| Genapol^{®} OX 030 | C_{12/15}-Alkylethoxylat mit 3 EO | C_{12/15}-Alkohol, wenig verzweigt |
| Genapol^{®} OX 050 | C_{12/15}-Alkylethoxylat mit 5 EO | C_{12/15}-Alkohol, wenig verzweigt |
| Genapol^{®} OX 070 | C_{12/15}-Alkylethoxylat mit 7 EO | C_{12/15}-Alkohol, wenig verzweigt |
| Genapol^{®} OX 080 | C_{12/15}-Alkylethoxylat mit 8 EO | C_{12/15}-Alkohol, wenig verzweigt |
| Genapol^{®} OX 100 | C_{12/15}-Alkylethoxylat mit 10 EO | C_{12/15}-Alkohol, wenig verzweigt |
| Genapol^{®} OX 109 | C_{12/15}-Alkylethoxylat mit 10 EO | C_{12/15}-Alkohol, wenig verzweigt |
| | | |
| Genapol^{®} OA 030 | C_{14/15}-Oxoalkohol mit 3 EO | C_{14/15}-Oxoalkohol |
| Genapol^{®} OA 3070 | C_{14/15}-Oxoalkohol, 3EO, 7PO | C_{14/15}-Oxoalkohol |
| Genapol^{®} OA 040 | C_{14/15}-Oxoalkohol mit 4 EO | C_{14/15}-Oxoalkohol |
| Genapol^{®} OA 050 | C_{14/15}-Oxoalkohol mit 5 EO | C_{14/15}-Oxoalkohol |
| Genapol^{®} OA 070 | C_{14/15}-Oxoalkohol mit 7 EO | C_{14/15}-Oxoalkohol |
| Genapol^{®} OA 080 | C_{14/15}-Oxoalkohol mit 8 EO | C_{14/15}-Oxoalkohol |
| | | |
| Hostacerin^{®} T3 | Cetylalkohol mit 3 EO | C₁₆-Alkohol, linear, gesättigt |
| | | |
| Hostacerin^{®} CS 200 | Cetylalkohol mit 20 EO | C₁₆-Alkohol, linear, gesättigt |
| Emulsogen^{®} HCO 040 | PEG-40 Rizinusöl, hydrogeniert | C_{16/18}-Hydroxyalkohol, linear, gesättigt |
| Emulsogen^{®} HCO 060 | PEG-60 Rizinusöl, hydrogeniert | C_{16/18}-Hydroxyalkohol, linear, gesättigt |

und
j) Alkoholalkoxylaten, EO/PO, insbesondere

| | | |
|---|---|---|
| Genapol^{®} EO 0244 | C_{10/12}-Alkohol mit 4 EO und 4 PO | C_{10/12}-Alkohol |
| Genapol^{®} EP 2464 | C_{12/14}-Alkohol mit 6 EO und 4 PO | C_{12/14}-Alkohol |
| Genapol^{®} EP 2544 | C_{12/15}-Oxoalkohol mit 4 EO und 4 PO | C_{12/15}-Oxoalkohol |
| Genapol^{®} EP 2564 | C_{12/15}-Oxoalkohol mit 6 EO und 4 PO | C_{12/15}-Oxoalkohol |
| Genapol^{®} EP 2584 | C_{12/15}-Oxoalkohol mit 8 EO und 4 PO | C_{12/15}-Oxoalkohol |
| Genapol^{®} EP2525 | C_{12/15}-Oxoalkohol mit 2 EO und 5 PO | C_{12/15}-Oxoalkohol |
| Genapol^{®} EP 2552 | C_{12/15}-Oxoalkohol mit 5 EO und 2 PO | C_{12/15}-Oxoalkohol |

sowie
(C₁₀-C₁₈)-Fettalkoholpolyglykolether mit 8 EO-Einheiten,
iso-(C₁₆-C₁₈)-Fettalkoholpolyglykolether mit 25 EO-Einheiten,
(C₁₈-C₂₂)-Fettalkoholpolyglykolether mit 25 EO-Einheiten,
Behenylalkoholether mit 10 EO-Einheiten,
Behenylalkoholether mit 20 EO-Einheiten und
Behenylalkoholether mit 25 EO-Einheiten
bevorzugt.

In der obigen Liste (Untergruppen a) bis j)) betreffend bevorzugte Acryl- und/oder Methacrysäureester von Alkoholpolyglykolethern bzw. von alkoxylierten Alkoholen gemäß Formel (Ib) sind links die Handelsnamen der alkoxylierten Alkohole, mittig die chemische Bezeichnung der alkoxylierten Alkohole und rechts eine Definition der Alkohole gegeben. Die Produkte stellen Handelsprodukte von Clariant dar. Im Rahmen der vorliegenden Erfindung bedeutet die Abkürzung "EO" Ethylenoxid -C₂H₄O- und "PO" Propylenoxid -C₃H₆O-.

In einer besonders bevorzugten Ausführungsform der Erfindung sind unter den Verbindungen der Formel (Ib) diejenigen bevorzugt, die ausgewählt sind aus Laurylmethacrylat, ethoxyliert mit 7 EO-Einheiten; Talylmethacrylat, ethoxyliert mit 8 EO-Einheiten; Talylmethacrylat, ethoxyliert mit 25 EO-Einheiten und Behenylmethacrylat, ethoxyliert mit 25 EO-Einheiten.

Im Rahmen der vorliegenden Erfindung wird die Bezeichnung "Talyl" synonym für die Bezeichnung "Talkalkyl" verwendet.

Außerordentlich bevorzugt sind die Verbindungen der Formel (Ia) ausgewählt aus Laurylacrylat, Laurylmethacrylat, Hexadecylacrylat, Hexadecylmethacrylat, Stearylacrylat, Stearylmethacrylat, Isostearylacrylat, Isotearylmethacrylat, Octyldodecylacrylat, Octyldodecylmethacrylat, Behenylacrylat und Behenylmethacrylat, und

die Verbindungen der Formel (Ib) ausgewählt aus Acrylsäureester von Laurylalkohol mit 7 EO-Einheiten, Methacrylsäureester von Laurylalkohol mit 7 EO-Einheiten, Acrylsäureester von Talylalkohol mit 8 EO-Einheiten, Methacrylsäureester von Talylalkohol mit 8 EO-Einheiten, Acrylsäureester von Talylalkohol mit 25 EO-Einheiten, Methacrylsäureester von Talylalkohol mit 25 EO-Einheiten, Acrylsäureester von Behenylalkohol mit 25 EO-Einheiten und Methacrylsäureester von Behenylalkohol mit 25 EO-Einheiten.

Darunter wiederum bevorzugt sind die Verbindungen der Formel (Ia) ausgewählt aus Laurylacrylat, Laurylmethacrylat, Hexadecylacrylat, Stearylacrylat, Isostearylacrylat, Octyldodecylacrylat und Behenylacrylat, und die Verbindungen der Formel (Ib) ausgewählt aus Methacrylsäureester von Laurylalkohol mit 7 EO-Einheiten, Methacrylsäureester von Talylalkohol mit 8 EO-Einheiten, Methacrylsäureester von Talylalkohol mit 25 EO-Einheiten und Methacrylsäureester von Behenylalkohol mit 25 EO-Einheiten.

In einer weiteren bevorzugten Ausführungsform der Erfindung bedeutet R² in den Verbindungen der Formel (I) oder in den Formeln (Ia) und (Ib) einen linearen oder verzweigten gesättigten Alkylrest mit 12 bis 200, bevorzugt mit 12 bis 58, besonders bevorzugt mit 12 bis 40 und insbesondere bevorzugt mit 12 bis 30 C-Atomen. In einer besonders bevorzugten Ausführungsform der Erfindung bedeutet R² in den Verbindungen der Formeln (I), (Ia) und (Ib) einen linearen gesättigten Alkylrest mit 12 bis 200, bevorzugt mit 12 bis 58, besonders bevorzugt mit 12 bis 40 und insbesondere bevorzugt mit 12 bis 30 C-Atomen.

Vorzugsweise sind die Struktureinheiten der Komponente b) hervorgegangen aus Substanzen ausgewählt aus
b1) polymerisierbaren quaternären Ammoniumverbindungen der folgenden Strukturformel (II)

   X⁻⁺NR⁷R⁸R⁹R¹⁰ (II)

   worin
   - X⁻: ein negativ geladenes Gegenion bedeutet,
   - R⁷: Vinyl-, Allyl- oder einen Rest der folgenden Strukturformel (III) 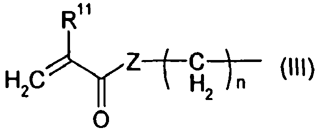
   bedeutet, worin
   - R¹¹: Wasserstoff oder Methyl bedeutet,
   - Z: O, NH, NCH₃ oder S bedeutet,
   - n: eine ganze Zahl von 1 bis 50 bedeutet,
   - R⁸: einen linearen oder verzweigten Alkylrest mit 1 bis 50 C-Atomen oder einen Rest R⁷ bedeutet,
   - R⁹: einen linearen oder verzweigten Alkylrest mit 1 bis 50 C-Atomen oder einen Rest R⁷ bedeutet und
   - R¹⁰: einen linearen oder verzweigten Alkylrest mit 1 bis 50 C-Atomen bedeutet
   und
b2) polymerisierbaren cyclischen quaternären Ammoniumverbindungen der folgenden Strukturformel (IV) 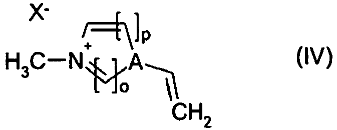 worin
   - o und p: jeweils unabhängig voneinander eine ganze Zahl von 0 bis 5 bedeuten,
   - A: C, CH, N oder P bedeutet und
   - X⁻: ein negativ geladenes Gegenion bedeutet.

In den Verbindungen der Formel (IV) ist in dem Fall, wenn o gleich Null oder geradzahlig ist, A gleich C und p ungeradzahlig.

In den Verbindungen der Formel (IV) kann p in dem Fall, dass o ungeradzahlig ist, Null, geradzahlig oder ungeradzahlig sein. Wenn p Null oder geradzahlig ist, ist A gleich C. Wenn p aber ungeradzahlig ist, kann A die Bedeutung CH, N oder P annehmen.

In den Verbindungen der Formel (IV) ist in dem Fall, wenn p gleich Null oder geradzahlig ist, A gleich C und o ungeradzahlig.

In den Verbindungen der Formel (IV) kann o in dem Fall, dass p ungeradzahlig ist, Null, geradzahlig oder ungeradzahlig sein. Wenn o Null oder geradzahlig ist, ist A gleich C. Wenn o aber ungeradzahlig ist, kann A die Bedeutung CH, N oder P annehmen.

Bevorzugte negativ geladene Gegenionen X- in Formel (II) und (IV) sind Chlorid, Bromid, Iodid, ½ SO₄²⁻ und Methosulfat.

In einer besonders bevorzugten Ausführungsform der Erfindung sind die Struktureinheiten der Komponente b) hervorgegangen aus polymerisierbaren quaternären Ammoniumverbindungen ausgewählt aus Diallyldimethylammoniumchlorid (DADMAC), [2-(Methacryloyloxy)ethyl]-trimethylammoniumchlorid (MAOETAC), [2-(Acryloyloxy)ethyl]-trimethylammoniumchlorid (AOETAC), [2-Methacrylamidoethyl]trimethylammoniumchlorid, [2-(Acrylamido)ethyl]trimethylammoniumchlorid, [3-Methacryl-amidopropyl]trimethylammoniumchlorid (MAPTAC), [3-Acrylamidopropyl]trimethylammoniumchlorid (APTAC), N-Methyl-2-vinylpyridiniumchlorid, N-Methyl-4-vinyl-pyridiniumchlorid und N-Methyl-3-vinylimidazoliniumchlorid.

In einer weiteren besonders bevorzugten Ausführungsform der Erfindung sind die Struktureinheiten der Komponente b) hervorgegangen aus polymerisierbaren quaternären Ammoniumverbindungen der oben genannten Strukturformel (II). Unter diesen Struktureinheiten der Komponente b) bevorzugt sind wiederum diejenigen, die hervorgegangen sind aus polymerisierbaren quaternären Ammoniumverbindungen der Strukturformel (II)

X⁻⁺NR⁷R⁸R⁹R¹⁰ (II)

worin
- X⁻: ein negativ geladenes Gegenion bedeutet,
- R⁷: Vinyl-, Allyl- oder einen Rest der folgenden Strukturformel (III) 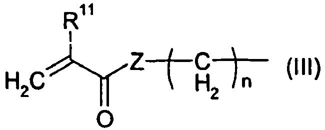
bedeutet, worin
- R¹¹: Wasserstoff oder Methyl bedeutet,
- Z: O, NH, NCH₃ oder S bedeutet,
- n: eine ganze Zahl von 1 bis 50 bedeutet,
- R⁸: einen linearen oder verzweigten Alkylrest mit 2 bis 50, bevorzugt 2 bis 25 und besonders bevorzugt 4 bis 8 C-Atomen oder einen Rest R⁷ bedeutet,
- R⁹: einen linearen oder verzweigten Alkylrest mit 1 bis 50, bevorzugt 2 bis 25 und besonders bevorzugt 4 bis 8 C-Atomen oder einen Rest R⁷ bedeutet und
- R¹⁰: einen linearen oder verzweigten Alkylrest mit 1 bis 50, bevorzugt 2 bis 25 und besonders bevorzugt 4 bis 8 C-Atomen, bedeutet.

In einer insbesondere bevorzugten Ausführungsform der Erfindung sind die Struktureinheiten der Komponente b) hervorgegangen aus polymerisierbaren quatemären Ammoniumverbindungen ausgewählt aus Diallyldimethylammoniumchlorid (DADMAC), [2-(Methacryloyloxy)ethyl]-trimethylammoniumchlorid (MAOETAC), [2-(Acryloyloxy)ethyl]-trimethylammoniumchlorid (AOETAC), [2-Methacrylamidoethyl]trimethylammoniumchlorid, [2-(Acrylamido)ethyl]trimethylammoniumchlorid, und [3-Meth-acrylamidopropyl]trimethylammoniumchlorid (MAPTAC) und [3-Acrylamidopropyl]-trimethylammoniumchlorid (APTAC).

In einer außerordentlich bevorzugten Ausführungsform der Erfindung enthalten die erfindungsgemäßen Polymere Struktureinheiten der Komponente b), die hervorgegangen sind aus 2-(Acryloyloxy)ethyl]trimethylammoniumchlorid. Diese Polymere sind gegenüber analogen Polymeren enthaltend Struktureinheiten hervorgegangen aus [3-Methacrylamidopropyl]trimethylammoniumchlorid bezüglich Klarheit und Transparenz in Ölgelen bevorzugt.

In einer weiteren bevorzugten Ausführungsform der Erfindung beträgt die Einsatzmenge an Substanzen ausgewählt aus Monomeren mit mehr als einer polymerisierbaren Gruppe, die vernetzend wirken können (Vernetzer), zur Herstellung der erfindungsgemäßen Copolymere, bezogen auf die Gesamtmasse der bei der Polymerisation zu polymerisierenden Monomere, 0 bis 20,0, bevorzugt 0 bis 10,0 und besonders bevorzugt 0 bis 5,0 Gew.-%.

Unter "polymerisierbarer Gruppe" wird hierbei eine polymerisierbare Doppelbindung verstanden.

In einer weiteren bevorzugten Ausführungsform der Erfindung enthalten die erfindungsgemäßen Copolymere eine oder mehrere Struktureinheiten der Komponente c), die hervorgegangen sind aus Substanzen ausgewählt aus Monomeren mit mehr als einer polymerisierbaren Gruppe, die vernetzend wirken können, in einer Menge von 0 bis 20,0, bevorzugt 0 bis 10,0 und besonders bevorzugt 0 bis 5,0 Gew.-%, bezogen auf die Gesamtmasse der erfindungsgemäßen Copolymere.

In einer besonders bevorzugten Ausführungsform der Erfindung enthalten die erfindungsgemäßen Copolymere keine Struktureinheiten der Komponente c), die hervorgegangen sind aus Substanzen ausgewählt aus Monomeren mit mehr als einer polymerisierbaren Gruppe, die vemetzend wirken können.

In einer weiteren besonders bevorzugten Ausführungsform der Erfindung enthalten die erfindungsgemäßen Copolymere ein oder mehrere Struktureinheiten der Komponente c), die hervorgegangen sind aus Substanzen ausgewählt aus Monomeren mit mehr als einer polymerisierbaren Gruppe, die vemetzend wirken können.

Vorzugsweise sind die vemetzenden Struktureinheiten der Komponente c) hervorgegangen aus Substanzen ausgewählt aus Divinylbenzol, Methylenbisacrylamid (MBA), Triallylamin, Triallylcyanurat, Ethandioldiacrylat, Ethandioldimethacrylat (EDDMA), Butandioldiacrylat, Butandioldimethacrylat (BDDMA), Hexandioldiacrylat, Hexandioldimethacrylat (HDDMA), Dodecandioldiacrylat, Dodecandioldimethacrylat (DDDMA), Trimethylolpropantriacrylat (TMPTA), Trimethylolpropantrimethacrylat (TMPTMA), Glycerindiacrylat, Glycerindimethacrylat, Tetraethylenglykoldiacrylat,
Tetraethylenglykoldimethacrylat, Poyethylenglykoldiacrylat mit Molmassen zwischen 400 und 800 g/mol, Poyethylenglykoldimethacrylat mit Molmassen zwischen 400 und 800 g/mol, Acryl-, Methacryl- oder Ethacrylsäureallylester, bevorzugt Acryl- oder Methacrylsäureallylester und besonders bevorzugt Methacrylsäureallylester (AMA), Dipropylenglykoldiallylether, Polyglykoldiallylether, Hydrochinondiallylether, Trimethylolpropandiallylether, Trimethylolpropantriallylether, Tetraallyloxyethan, Triethylenglykoldivinylether und anderen Allyl- oder Vinylethem multifunktioneller Alkohole, sowie aus Mischungen der vorgenannten Substanzen, wie z. B. aus Mischungen aus Hexandioldimethacrylat und Trimethylolpropantrimethacrylat.

Darunter wiederum bevorzugt sind die vernetzenden Struktureinheiten der Komponente c) hervorgegangen aus Substanzen ausgewählt aus Methylenbisacrylamid (MBA), Triallylamin, Triallylcyanurat, Ethandioldimethacrylat (EDDMA), Butandioldimethacrylat (BDDMA), Hexandioldimethacrylat (HDDMA), Dodecandioldimethacrylat (DDDMA), Trimethylolpropantriacrylat (TMPTA), Trimethylolpropantrimethacrylat (TMPTMA), Glycerindiacrylat, Poyethylenglykoldiacrylat mit Molmassen zwischen 400 und 800 g/mol, Methacrylsäureallylester (AMA), sowie aus Mischungen der vorgenannten Substanzen, wie z. B. aus Mischungen aus Hexandioldimethacrylat und Trimethylolpropantrimethacrylat.

Besonders bevorzugt sind die vernetzenden Struktureinheiten der Komponente c) hervorgegangen aus Hexandioldimethacrylat, Trimethylolpropantriacrylat und Mischungen aus diesen Substanzen.

In einer insbesondere bevorzugten Ausführungsform der Erfindung beträgt die Einsatzmenge des Vernetzers zur Herstellung der erfindungsgemäßen Copolymere, bezogen auf die Gesamtmasse der bei der Polymerisation zu polymerisierenden Monomere, 0,1 bis 20,0, bevorzugt 0,5 bis 10,0 und besonders bevorzugt 1,0 bis 5,0 Gew.-%.

Sofern die erfindungsgemäßen Copolymere ein oder mehrere Struktureinheiten der Komponente c) enthalten, die hervorgegangen sind aus Substanzen ausgewählt aus Monomeren mit mehr als einer polymerisierbaren Gruppe, die vernetzend wirken können, enthalten sie in einer weiteren insbesondere bevorzugten Ausführungsform der Erfindung 0,1 bis 20,0, bevorzugt 0,5 bis 10,0 und besonders bevorzugt 1,0 bis 5,0 Gew.-% an diesen Struktureinheiten, bezogen auf die Gesamtmasse der erfindungsgemäßen Copolymere.

In einer weiteren bevorzugten Ausführungsform der Erfindung enthalten die Copolymere eine oder mehrere Struktureinheiten der Komponente c), die hervorgegangen sind aus Substanzen ausgewählt aus Acrylsäure- oder Methacrylsäureestem von Alkylalkoholen mit 1 bis 5 Kohlenstoffatomen, die gegebenenfalls auch mit 1 bis 25 Alkylenoxideinheiten wie z. B. mit Ethylenoxideinheiten, mit Propylenoxideinheiten oder mit Mischungen aus Ethylenoxideinheiten und Propylenoxideinheiten alkoxyliert sein können, wobei unter diesen Verbindungen Butylacrylat bevorzugt ist, Hydroxyethylmethacrylat (HEMA), (Meth)Acrylsäureestern von Polyethylenglykolen, Polypropylenglykolen und Polyethylen-polypropylenglykolen, N-Vinylpyrrolidon sowie Ethen, Propen, 1-Buten und 1-Penten. Unter den genannten Verbindungen ist Butylacrylat bevorzugt. Sofern die erfindungsgemäßen Copolymere eine oder mehrere der soeben genannten Struktureinheiten enthalten, sind diese vorzugsweise in einer Menge von 0,1 bis 10,0 Gew.-%, bezogen auf die Gesamtmasse des Copolymers, in den erfindungsgemäßen Copolymeren enthalten.

Die erfindungsgemäßen Copolymere können z. B. auch "gegrafted" sein, d. h. auf einem Träger aufgebracht sein. Die Herstellung von Trägerpolymeren ist allgemein bekannt. Sofern ein Träger verwendet wird, ist die Menge des Trägers, bezogen auf die Masse des geträgerten Copolymers, vorzugsweise von 0,1 bis 10,0 Gew.-% und besonders bevorzugt von 2,0 bis 6,0 Gew.-%. Sofern ein Träger verwendet wird, ist dieser vorzugsweise Polyvinylpyrrolidon, Polyethylenglykol, Polypropylenglykol oder Polyethylen/polypropylenglykol und besonders bevorzugt Polyvinylpyrrolidon.

Bevorzugt ist in den erfindungsgemäßen Copolymeren die Menge an Struktureinheiten der Komponente a) von 60,0 bis 99,5 Gew.-%, die Menge an Struktureinheiten der Komponente b) von 0,5 bis 20,0 Gew.-% und die Menge an Struktureinheiten der Komponente c) von 0 bis 20,0 Gew.-%.

Besonders bevorzugt ist in den erfindungsgemäßen Copolymeren die Menge an Struktureinheiten der Komponente a) von 75,0 bis 98,5 Gew.-%, die Menge an Struktureinheiten der Komponente b) von 0,6 bis 12,5 Gew.-% und die Menge an Struktureinheiten der Komponente c) von 0 bis 12,5 Gew.-%.

Insbesondere bevorzugt ist in den erfindungsgemäßen Copolymeren die Menge an Struktureinheiten der Komponente a) von 80,0 bis 98,0 Gew.-%, die Menge an Struktureinheiten der Komponente b) von 0,7 bis 11,0 Gew.-% und die Menge an Struktureinheiten der Komponente c) von 0 bis 11,0 Gew.-%.

Das Gewichtsverhältnis an Struktureinheiten hervorgegangen aus den Verbindungen der Formel (Ia) : Struktureinheiten hervorgegangen aus den Verbindungen der Formel (Ib) in den erfindungsgemäßen Copolymeren ist vorzugsweise 1,0 : 0 bis 0,8.

In einer weiteren bevorzugten Ausführungsform der Erfindung enthalten die erfindungsgemäßen Copolymere keine Struktureinheiten, die aus anionischen polymerisierbaren Substanzen hervorgegangen sind.

In einer weiteren bevorzugten Ausführungsform der Erfindung bestehen die erfindungsgemäßen Copolymere aus den unter den Komponenten a) und b) genannten Struktureinheiten.

In einer weiteren bevorzugten Ausführungsform der Erfindung bestehen die erfindungsgemäßen Copolymere aus den unter den Komponenten a), b) und c) genannten Struktureinheiten. Unter diesen Copolymeren sind wiederum diejenigen bevorzugt, worin die Struktureinheiten der Komponente c) aus vernetzenden Struktureinheiten ausgewählt sind.

In einer besonders bevorzugten Ausführungsform der Erfindung enthalten die erfindungsgemäßen Copolymere von 95,0 bis 99,0 Gew.% an einer oder mehreren Struktureinheiten hervorgegangen aus den Verbindungen der Formel (Ia) gemäß Komponente a), vorzugsweise ausgewählt aus Stearylacrylat und Laurylmethacrylat, und von 0,1 bis 5,0 Gew.-% an einer oder mehreren Struktureinheiten hervorgegangen aus den Verbindungen der Komponente b), vorzugsweise [2-(Acryloyloxy)-ethyl]trimethylammonium-chlorid (AOETAC) (im Folgenden Copolymere A).

Unter den Copolymeren A ist das Copolymer bestehend aus 97,0 Gew.-% an Struktureinheiten hervorgegangen aus Stearylacrylat und 3,0 Gew.% an Struktureinheiten hervorgegangen aus [2-(Acryloyloxy)-ethyl]trimethylammoniumchlorid (AOETAC) bevorzugt (z. B. Copolymere Nr. 1, 273 und 278).

Unter den Copolymeren A ist weiterhin das Copolymer bestehend aus 82,0 Gew.% an Struktureinheiten hervorgegangen aus Stearylacrylat, 15,0 Gew.-% an Struktureinheiten hervorgegangen aus Laurylmethacrylat und 3,0 Gew.-% an Struktureinheiten hervorgegangen aus [2-(Acryloyloxy)-ethyl]trimethylammoniumchlorid (AOETAC) bevorzugt (z. B. Copolymer Nr. 277).

In einer anderen besonders bevorzugten Ausführungsform der Erfindung enthalten die erfindungsgemäßen Copolymere von 90,0 bis 98,0 Gew.-% an einer oder mehreren Struktureinheiten hervorgegangen aus Verbindungen der Formel (I) gemäß Komponente a), vorzugsweise ausgewählt aus Stearyacrylat, Isostearylacrylat und Behenylmethacrylat, ethoxyliert mit 25 EO-Einheiten (B250MA) von 1,5 bis 5,0 Gew.-% an einer oder mehreren Struktureinheiten hervorgegangen aus Verbindungen der Komponente b), vorzugsweise ausgewählt aus [2-(Acryloyloxy)-ethyl]trimethylammoniumchlorid (AOETAC) und [3-Methacryl-amidopropyl]trimethylammoniumchlorid (MAPTAC) und von 0,5 bis 6,0 Gew.-% an einer oder mehreren Struktureinheiten hervorgegangen aus Verbindungen der Komponente c), vorzugsweise ausgewählt aus Vernetzern, besonders bevorzugt ausgewählt aus Hexandioldimethacrylat (HDDMA) und Trimethylolpropantriacrylat (TMPTA) (im Folgenden Copolymere B).

Unter den Copolymeren B sind diejenigen bevorzugt, die als Struktureinheiten hervorgegangen aus Verbindungen der Formel (I) gemäß Komponente (a) Struktureinheiten hervorgegangen aus Verbindungen der Formeln (Ia) und (Ib) enthalten, und diese wiederum bevorzugt in dem Gewichtsverhältnis Struktureinheiten hervorgegangen aus der oder den Verbindungen der Formel (Ia) : Struktureinheiten hervorgegangen aus der oder den Verbindungen der Formel (Ib) = 1,0 : 0,1 bis 1,0 (im Folgenden Copolymere B1).

Unter den Copolymeren B1 sind die Copolymere bestehend aus 56,4 Gew.-% an Struktureinheiten hervorgegangen aus Stearylacrylat, 39,6 Gew.% an Struktureinheiten hervorgegangen aus Behenylmethacrylat, ethoxyliert mit 25 EO-Einheiten (B250MA), 3,0 Gew.-% an Struktureinheiten hervorgegangen aus [2-(Acryloyloxy)-ethyl]trimethylammoniumchlorid (AOETAC) und 1,0 Gew.-% an Struktureinheiten hervorgegangen aus Hexandioldimethacrylat (HDDMA) bevorzugt (z. B. Copolymer Nr. 30).

Unter den Copolymeren B1 sind weiterhin diejenigen Copolymere bestehend aus 74,6 Gew.-% an Struktureinheiten hervorgegangen aus Stearylacrylat, 9,5 Gew.-% an Struktureinheiten hervorgegangen aus Isostearylacrylat, 8,6 Gew.% an Struktureinheiten hervorgegangen aus Behenylmethacrylat, ethoxyliert mit 25 EO-Einheiten (B250MA), 2,5 Gew.-% an Struktureinheiten hervorgegangen aus [2-(Acryloyloxy)-ethyl]trimethylammoniumchlorid (AOETAC) und 4,8 Gew.-% an Struktureinheiten hervorgegangen aus Hexandioldimethacrylat (HDDMA) bevorzugt (z. B. Copolymer Nr. 40).

Auch Copolymere bestehend aus 78,2 Gew.-% an Struktureinheiten hervorgegangen aus Stearylacrylat, 9,0 Gew.-% an Struktureinheiten hervorgegangen aus Behenylmethacrylat, ethoxyliert mit 25 EO-Einheiten (B250MA), 4,7 Gew.-% an Struktureinheiten hervorgegangen aus C₂₆-C₂₈ α-Olefin, 3,6 Gew.-% an Struktureinheiten hervorgegangen aus einer Mischung aus [2-(Acryloyloxy)-ethyl]trimethylammoniumchlorid (AOETAC) und [3-Methacryl-amidopropyl]trimethylammoniumchlorid (MAPTAC) und 4,7 Gew.-% an Struktureinheiten hervorgegangen aus einer Mischung aus Hexandioldimethacrylat (HDDMA) und Trimethylolpropantriacrylat (TMPTA) sind bevorzugt (z. B. Copolymer Nr. 111).

Weiterhin sind Copolymere bestehend aus 78,2 Gew.-% an Struktureinheiten hervorgegangen aus Stearylacrylat, 9,0 Gew.-% an Struktureinheiten hervorgegangen aus Behenylmethacrylat, ethoxyliert mit 25 EO-Einheiten (B250MA), 4,7 Gew.-% an Struktureinheiten hervorgegangen aus C₂₆-C₂₈ α-Olefin, 3,6 Gew.-% an Struktureinheiten hervorgegangen aus [2-(Acryloyloxy)-ethyl]trimethylammoniumchlorid (AOETAC) und 4,7 Gew.-% an Struktureinheiten hervorgegangen aus einer Mischung aus Hexandioldimethacrylat (HDDMA) und Trimethylolpropantriacrylat (TMPTA) bevorzugt (z. B. Copolymer Nr. 60).

Auch Copolymere bestehend aus 84,1 Gew.% an Struktureinheiten hervorgegangen aus Stearylacrylat, 9,4 Gew.-% an Struktureinheiten hervorgegangen aus Behenylmethacrylat, ethoxyliert mit 25 EO-Einheiten (B250MA), 1,6 Gew.-% an Struktureinheiten hervorgegangen aus C₂₆-C₂₈ α-Olefin, 1,6 Gew.% an Struktureinheiten hervorgegangen aus [2-(Acryloyloxy)-ethyl]trimethylammoniumchlorid (AOETAC) und 3,3 Gew.-% an Struktureinheiten hervorgegangen aus Trimethylolpropantriacrylat (TMPTA) sind bevorzugt (z. B. Copolymer Nr. 52).

Die Verteilung der verschiedenen Struktureinheiten in den erfindungsgemäßen Copolymeren kann statistisch, blockartig, alternierend oder gradientenartig sein.

Die erfindungsgemäßen Copolymere besitzen bevorzugt ein Molekulargewicht von 10³ bis 10⁹ g/mol, besonders bevorzugt von 10⁴ bis 10⁷ g/mol und insbesondere bevorzugt von 5*10⁵ bis 5*10⁶ g/mol.

Die Herstellung der erfindungsgemäßen Copolymere erfolgt durch radikalische Polymerisationsreaktion der polymerisierbaren Substanzen aus denen die wiederkehrenden Struktureinheiten der Komponenten a), b) und gegebenenfalls c) sowie gegebenenfalls weitere Struktureinheiten hervorgehen.

Ein weiterer Gegenstand der Erfindung ist ein Verfahren zur Herstellung eines erfindungsgemäßen Copolymers dadurch gekennzeichnet, dass die Monomere in einer radikalischen Polymerisationsreaktion polymerisiert werden.

Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt Verwendung finden organische Lösungsmittel, insbesondere niedere, tertiäre Alkohole oder Kohlenwasserstoffe mit 3 bis 30 C-Atomen. In einer besonders bevorzugten Ausführungsfrom wird tert.-Butanol oder Toluol als Reaktionsmedium verwendet. Mischungen aus zwei- oder mehreren Vertretern der beschriebenen potentiellen Lösungsmittel sind selbstverständlich ebenfalls erfindungsgemäß. Dies schließt auch Emulsionen von nicht miteinander mischbaren Solventien ein (z. B. Wasser/Kohlenwasserstoffe). Grundsätzlich sind alle Arten der Reaktionsführung geeignet, die zu den erfindungsgemäßen Polymerstrukturen führen (Lösungspolymerisation, Emulsionsverfahren, Fällungsverfahren, Hochdruckverfahren, Suspensionsverfahren, Substanzpolymerisation, Gelpolymerisation usw.).

Die Polymerisationsreaktion erfolgt bevorzugt im Temperaturbereich zwischen 0 und 150 °C, besonders bevorzugt zwischen 10 und 110 °C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff oder Argon, ausgeführt werden.

Zur Auslösung der Polymerisation können energiereiche elektromagnetische Strahlen, mechanische Energie oder die üblichen chemischen Polymerisationsinitiatoren, wie organische Peroxide, z. B. Benzoylperoxid, tert.-Butylhydroperoxid, Methylethylketonperoxid, Cumolhydroperoxid, Dilauroylperoxid oder Azoinitiatoren, wie z. B. Azodiisobutyronitril (AIBN), Azomethylbutyronitril (AMBN) oder Dimethyl-2,2'-azobis(2-methylpropionat), verwendet werden.

Bei der Herstellung der erfindungsgemäßen Copolymere können auch Substanzen verwendet werden, die die Polymerisation bzw. die Kettenlänge der erfindungsgemäßen Copolymere regeln, wie z. B. Methallylsulfonat, Isopropanol oder Dodecylmercaptan. Die Verwendung von derartigen Substanzen bei Polymerisationsreaktionen ist allgemein bekannt. Bei Verwendung eines Initiators für die Polymerisationsreaktion gilt entsprechendes.

Bei der Herstellung der erfindungsgemäßen Copolymere können auch Substanzen verwendet werden, die die radikalische Polymerisation durch eine reversible Deaktivierung der sehr reaktiven Kettenenden kontrollieren. Sofern die kontrollierte radikalische Polymerisation zum Einsatz kommt, wird sie vorzugsweise mit Organohalogeniden / Übergangsmetallkomplexverbindungen, bevorzugt sekundären Alkylhalogeniden in Verbindung mit Kupfer (I) Komplexen als ATRP (Atom Transfer Radikal Polymerisation) oder durch die Verwendung von linearen oder cyclischen Nitroxiden, bevorzugt als sogenannte TEMPO-Methode durchgeführt.

Eine bevorzugte Ausführungsform des erfindungsgemäßen Verfahrens ist dadurch gekennzeichnet, dass die radikalische Polymerisation in einem organischen Lösungsmittel, bevorzugt in tert.-Butanol oder Toluol, besonders bevorzugt in tert.-Butanol, erfolgt.

Eine weitere bevorzugte Ausführungsform des erfindungsgemäßen Verfahrens ist dadurch gekennzeichnet, dass die Isolierung der erfindungsgmäßen Copolymere durch Abdestillieren des Lösungsmittels und durch anschließende Vakuumtrocknung erfolgt.

Eine weitere bevorzugte Ausführungsform der Erfindung ist dadurch gekennzeichnet, dass die Isolierung der erfindungsgemäßen Copolymere durch Fällung in einem non-solvent, d. h. einem Lösungsmittel, in dem das erfindungsgemäße Copolymer unlöslich ist, bevorzugt in Wasser, und durch anschließende Filtration und Trocknung im Vakuum erfolgt.

Die oben beschriebenen Copolymere sind geruchsneutral, weiß bis beige und haben ausgezeichnete Verarbeitungseigenschaften; sie sind hautfreundlich, gut kompatibel mit allen gängigen Bestandteilen kosmetischer Formulierungen und daher zur Herstellung kosmetischer, dermatologischer und pharmazeutischer Formulierungen geeignet.

Ein weiterer Gegenstand der Erfindung ist daher eine kosmetische, pharmazeutische oder dermatologische Formulierung, vorzugsweise eine kosmetische Formulierung, dadurch gekennzeichnet, dass sie ein oder mehrere erfindungsgemäße Copolymere enthält.

In einer bevorzugten Ausführungsform der Erfindung sind die erfindungsgemäßen kosmetischen Formulierungen Hautreinigunsmittel, Hautpflegeprodukte oder Haarbehandlungsmittel.

Die erfindungsgemäßen Copolymere zeichnen sich durch ein ausgezeichnetes Ölbindevermögen aus und sind hervorragend geeignet zum Verdicken von Formulierungen auf Öl-Basis.

Ein weiterer Gegenstand der Erfindung ist daher die Verwendung eines oder mehrerer der erfindungsgemäßen Copolymere zur Verdickung von Ölen. Bei diesen Ölen handelt es sich vorzugsweise um kosmetische Öle, d. h. um Öle, die in kosmetischen Formulierungen Verwendung finden können.

Die Öle können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Methanol, Isopropanol, Propylenglykol oder Glycerin, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318 sowie das Handelsprodukt Velsan^{®} CCT (Capryl-Caprinsäure-triglycerid, Clariant). Auch gehärtete Triglyceride sind erfindungsgemäß bevorzugt. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten C₈₋₂₂-Alkanolen, z.B. die Handelsprodukte Finsolv^{®}SB (Isostearylbenzoat), Finsolv^{®}TN (C₁₂-C₁₅-Alkylbenzoat) und Finsolv^{®}EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether, Di-3-ethyldecylether, tert.-Butyl-n-octylether, iso-Pentyl-n-octylether und 2-Methyl-pentyl-n-octylether sowie Di-tert.-butylether und Di-iso-pentylether.

Ebenso in Betracht kommen verzweigte gesättigte oder ungesättigte Fettalkohole mit 6-30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z. B. unter dem Handelsnamen Cosmacol^{®} der EniChem, Augusta Industriale.

Eine weitere Klasse von bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®} S), Ozokerit, und Ceresin.

Ebenso in Betracht kommen Silikonöle bzw. -wachse, vorzugsweise Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare^{®} Silicone 41 M65, SilCare^{®} Silicone 41 M70, SilCare^{®} Silicone 41 M80 (Clariant) erhältlichen Dimethicone, Stearyldimethylpolysiloxan, C₂₀-C₂₄-Alkyl-dimethylpolysiloxan, C₂₄-C₂₈-Alkyl-dimethylpolysiloxan, aber auch die unter SilCare^{®} Silicone 41 M40, SilCare^{®} Silicone 41 M50 (Clariant) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die erfindungsgemäßen Copolymere können auch in vorteilhafter Weise zur Herstellung von Ölgelen verwendet werden. In einer weiteren bevorzugten Ausführungsform der Erfindung sind die erfindungsgemäßen kosmetischen, pharmazeutischen oder dermatologischen Formulierungen daher Ölgele wie beispielsweise verdickte Babyöle.

Die erfindungsgemäßen Ölgele enthalten vorzugsweise 90,0 bis 99,99 Gew.-%, besonders bevorzugt 94,0 bis 99,9 Gew.-% und insbesondere bevorzugt 97,0 bis 99,5 Gew.-% an einem oder mehreren Ölen oder Ölkörpern und vorzugsweise 0,01 bis 10,0 Gew.-%, besonders bevorzugt 0,1 bis 6,0 Gew.-% und insbesondere bevorzugt 0,5 bis 3,0 Gew.% an einem oder mehreren der erfindungsgemäßen Copolymere, jeweils bezogen auf das gesamte Ölgel. Die vorzugsweise in den erfindungsgemäßen Ölgelen enthaltenen Öle oder Ölkörper entsprechen den zuvor genannten bevorzugten Ölen oder Ölkörpem.

Weiterhin vorteilhaft sind die hervorragenden konditionierenden Eigenschaften der erfindungsgemäßen Copolymere gegenüber keratinischen Fasern. Sie bewirken ein ausgezeichnetes Hautgefühl und sind den wachshaltigen Formulierungen überlegen, die häufig ein klebriges und stumpfes Verhalten zeigen. Sie verbessern den Glanz der Haare, sowie die leichte Kämmbarkeit der Haare.

Weiterer Gegenstand der vorliegenden Erfindung ist daher die Verwendung eines oder mehrerer der erfindungsgemäßen Copolymere zur Konditionierung von Fasern, vorzugsweise von keratinischen Fasern, besonders bevorzugt in kosmetischen Formulierungen.

In einer besonders bevorzugten Ausführungsform der Erfindung sind die erfindungsgemäßen kosmetischen Formulierungen Haarbehandlungsmittel. Bevorzugt sind hierbei Haarbehandlungsmittel, insbesondere Haarkonditioniermittel wie Cremespülungen und Haarkuren, die zur Verbesserung des Glanzes, der Kämmbarkeit und zum Hitzeschutz der Haare eingesetzt werden. In einer insbesondere bevorzugten Ausführungsform der Erfindung enthalten die erfindungsgemäßen Haarbehandlungsmittel, insbesondere die soeben genannten Haarbehandlungsmittel, ein oder mehrere erfindungsgemäße Copolymere und ein oder mehrere weitere kationische Tenside wie Cetrimoniumchlorid, Behentrimoniumchlorid, Stearamidopropyldimethylamin, Behenamidopropyldimethylamin oder quartäre Ammoniumverbindungen auf Esterbasis und/oder ein oder mehrere Silikone.

Wegen ihrer Substantivität und geringen Wasserlöslichkeit eignen sich die erfindungsgemäßen Copolymere für die Anwendung als Wasserschutzverbesserer. Weiterer Gegenstand der Erfindung ist daher die Verwendung eines oder mehrerer der erfindungsgemäßen Polymere als Wasserschutzverbesserer.

Die erfindungsgemäßen Copolymere sind außerdem zur Oberflächenbehandlung von Pigmenten wie z. B. FeO, TiO₂ und ZnO geeignet. Weiterer Gegenstand der Erfindung ist daher die Verwendung eines oder mehrerer der erfindungsgemäßen Polymere zur Oberflächenbehandlung von Pigmenten.

Die erfindungsgemäßen Copolymere sind außerdem in vorteilhafter Weise zur Erhöhung der Wasserfestigkeit von Sonnenschutzformulierungen geeignet.

Weiterer Gegenstand der Erfindung ist daher die Verwendung eines oder mehrerer der erfindungsgemäßen Copolymere zur Erhöhung der Wasserfestigkeit von Sonnenschutzformulierungen.

Des Weiteren sind die erfindungsgemäßen Copolymere in vorteilhafter Weise zur Erhöhung des Sonnenschutzfaktors in Sonnenschutzformulierungen geeignet.

Weiterer Gegenstand der Erfindung ist daher die Verwendung eines oder mehrerer der erfindungsgemäßen Copolymere zur Erhöhung des Sonnenschutzfaktors von Sonnenschutzformulierungen.

In einer weiteren bevorzugten Ausführungsform der Erfindung sind die erfindungsgemäßen Formulierungen Sonnenschutzmittel und enthalten einen oder mehrere anorganische und/oder organische Sonnenschutzfilter.

Wegen der pigmentdispergierenden Eigenschaften der erfindungsgemäßen Copolymere sind die erfindungsgemäßen kosmetischen Formulierungen oder Mittel in einer weiteren bevorzugten Ausführungsform der Erfindung dekorative Kosmetika wie z. B. Puder, Foundations, Mascara oder Lippenstifte.

Die erfindungsgemäßen Copolymere sind außerdem in vorteilhafter Weise als Emulgator, insbesondere in kosmetischen Formulierungen, geeignet.

Weiterer Gegenstand der Erfindung ist daher die Verwendung eines oder mehrerer der erfindungsgemäßen Copolymere als Emulgator, vorzugsweise in kosmetischen Formulierungen.

In einer weiteren bevorzugten Ausführungsform der Erfindung liegen die erfindungsgemäßen Formulierungen als Emulsionen vom Typ Wasser-in-Öl oder Öl-in-Wasser vor, insbesondere die erfindungsgemäßen Hautpflegeprodukte. In einer besonders bevorzugten Ausführungsform der Erfindung enthalten diese Emulsionen neben dem einen oder den mehreren erfindungsgemäßen Copolymeren ein oder mehrere nichtionische Emulgatoren. Unter den Emulsionen sind die Emulsionen vom Typ Wasser-in-Öl bevorzugt.

Eine weitere bevorzugte Ausführungsform der Erfindung ist die Verwendung eines oder mehrerer der Copolymere in Formulierungen, die frei sind von Wachskomponenten.

Die erfindungsgemäßen Copolymere sind sowohl als Verdicker, Konsistenzgeber und Sensorikadditiv, als auch als Emulgator, Solubilisator, Dispergator, Suspendiermittel, Gleitmittel, Haftmittel und Stabilisator geeignet.

Ein weiterer Gegenstand der Erfindung betrifft daher die Verwendung eines oder mehrerer der erfindungsgemäßen Copolymere als Verdicker, Konsistenzgeber, Sensorikadditiv, Emulgator, Solubilisator, Dispergator, Suspendiermittel, Gleitmittel, Haftmittel oder Stabilisator.

Die erfindungsgemäßen Formulierungen enthalten, bezogen auf die fertigen kosmetischen, pharmazeutischen oder dermatologischen Formulierungen, vorzugsweise 0,01 bis 10,0 Gew.-%, besonders bevorzugt 0,1 bis 6,0 Gew.% und insbesondere bevorzugt 0,5 bis 3,0 Gew.-% an den erfindungsgemäßen Copolymeren.

Die erfindungsgemäßen Formulierungen können kationische, nichtionische, ampholytische Tenside, Betaintenside und/oder in geringen Mengen anionische Tenside enthalten.

Die Gesamtmenge der in den erfindungsgemäßen Mitteln (z. B. im Falle von Rinse-Off-Produkten) eingesetzten Tenside beträgt, bezogen auf die fertigen erfindungsgemäßen Formulierungen, bevorzugt von 1,0 bis 70,0 Gew.-%, besonders bevorzugt von 5,0 bis 40,0 Gew.-% und insbesondere bevorzugt von 10,0 bis 35,0 Gew.-%.

Als anionische Tenside bevorzugt sind (C₁₀-C₂₂)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate; Acylester von Isethionaten, α-Sulfofettsäureester, Alkylbenzolsulfonate, Alkylphenolglykolethersulfonate, Sulfosuccinate, Sulfobemsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate und Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Die Menge der anionischen Tenside in den erfindungsgemäßen Formulierungen beträgt bevorzugt von 0,1 bis 10,0 Gew.%, besonders bevorzugt von 0,2 bis 5,0 Gew.-% und insbesondere bevorzugt von 0,5 bis 2,0 Gew.-%, bezogen auf die fertigen Formulierungen.

Bevorzugte kationische Tenside sind quartäre Ammonium-Salze, wie Di-(C₈-C₂₂)-Alkyl-dimethylammoniumchlorid oder -bromid, vorzugsweise Di-(C₈-C₂₂)-Alkyl-dimethylammoniumchlorid oder -bromid; (C₈-C₂₂)-Alkyl-dimethyl-ethylammoniumchlorid oder -bromid; (C₈-C₂₂)-Alkyl-trimethylammoniumchlorid oder -bromid, vorzugsweise Cetyltrimethylammoniumchlorid oder -bromid und (C₈-C₂₂)-Alkyl-trimethylammoniumchlorid oder -bromid; (C₁₀-C₂₄)-Alkyl-dimethylbenzyl-ammoniumchlorid oder -bromid, vorzugsweise (C₁₂-C₁₈)-Alkyl-dimethylbenzyl-ammoniumchlorid, (C₈-C₂₂)-Alkyl-dimethyl-hydroxyethylammoniumchlorid, -phosphat, -sulfat, -lactat, (C₈-C₂₂)-Alkylamidopropyltrimethylammoniumchlorid, -methosulfat, N,N-bis(2-C₈-C₂₂-Alkanoyl-oxyethyl)-dimethylammoniumchlorid, -methosulfat, N,N-bis(2-C₈-C₂₂-Alkanoyl-oxyethyl)hydroxyethyl-methyl-ammoniumchlorid, -methosulfat.

Die Menge der kationischen Tenside in den erfindungsgemäßen Formulierungen beträgt bevorzugt von 0,1 bis 10,0 Gew.-%, besonders bevorzugt von 0,5 bis 7,0 Gew.-% und insbesondere bevorzugt von 1,0 bis 5,0 Gew.-%, bezogen auf die fertigen Formulierungen.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkanolamide, (Fettsäureamidpolyethylenglykole); Saccharoseester; Sorbitester und Sorbitanester und deren Polyglykolether, sowie C₈-C₂₂-Alkylpolyglucoside.

Die Menge der nichtionischen Tenside in den erfindungsgemäßen Formulierungen (z. B. im Falle von Rinse-off-Produkten) liegt bevorzugt im Bereich von 1,0 bis 20,0 Gew.%, besonders bevorzugt von 2,0 bis 10,0 Gew.% und insbesondere bevorzugt von 3,0 bis 7,0 Gew.-%, bezogen auf die fertigen Formulierungen.

Weiterhin können die erfindungsgemäßen Formulierungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide, Fettsäureamidoalkyl-dimethylaminoxid.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Kokosacylamidopropyldimethylbetain oder die C₁₂- bis C₁₈-Dimethylaminohexanoate bzw. die C₁₀- bis C₁₈-Acylamidopropandimetylbetaine.

Die Menge der amphoteren Tenside und/oder Betaintenside in den erfindungsgemäßen Formulierungen beträgt bevorzugt von 0,5 bis 20,0 Gew.-% und besonders bevorzugt von 1,0 bis 10,0 Gew.-%, bezogen auf die fertigen Formulierungen.

Bevorzugte Tenside sind Laurylsulfat, Laurethsulfat, Cocoamidopropylbetain, Alkylbetaine wie Coco-Betain, Natriumcocoylglutamat und Lauroamphoacetat.

In einer weiteren bevorzugten Ausführungsform der Erfindung enthalten die erfindungsgemäßen Formulierungen zusätzlich noch als schaumverstärkende Mittel Co-Tenside aus der Gruppe der Alkylbetaine, Alkylamidobetaine, Aminopropionate, Aminoglycinate, Imidazoliniumbetaine und Sulfobetaine, Aminoxide, Fettsäurealkanolamide und Polyhydroxyamide.

Die erfindungsgemäßen Formulierungen können als weitere Hilfs- und Zusatzstoffe, Wachse, Emulgatoren, Co-Emulgatoren, Solubilisatoren, Elektrolyte, Hydroxysäuren, Stabilisatoren, weitere kationische Polymere, Filmbildner, weitere Verdicker, Gelierungsmittel, Überfettungsmittel, Rückfetter, antimikrobielle Wirkstoffe, biogene Wirkstoffe, Adstringentien, deodorierende Mittel, Sonnenschutzfilter, Antioxidantien, Feuchthaltemittel, Lösungsmittel, Farbmittel, Duftstoffe, Perlglanzmittel, Trübungsmittel und/oder wasserlösliche Silikone enthalten.

Die erfindungsgemäßen Formulierungen können Wachse, beispielsweise Paraffinwachse, Mikrowachse und Ozokerite, Bienenwachs und ihre Teilfraktionen sowie der Bienenwachsderivate, Wachse aus der Gruppe der homopolymeren Polyethylene oder Coplymere der α-Olefine, sowie natürliche Wachse wie Reiswachs, Candellilawachs, Camaubawachs, Japanwachs oder Schellackwachs enthalten.

Als Emulgatoren, Co-Emulgatoren und Solubilisatoren können nichtionische, anionische, kationische oder amphotere oberflächenaktive Verbindungen eingesetzt werden.

Als nichtionogene oberflächenaktive Verbindungen kommen vorzugsweise in Betracht:
Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono-und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z.B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole und Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethylenglycol(19)stearylether, Polyethylenglycol(20)stearylether, Polyethylenglycol(12)isostearylether, Polyethylengtycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Pofyethylenglycol(15)isocetylether, Potyethylengtycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether.

Ebenso bevorzugt sind Fettsäureethoxylate, gewählt aus der Gruppe der ethoxylierten Stearate, Isostearate und Oleate, insbesondere
Polyethylenglycol(20)stearat, Polyethylenglykol(21 )stearat,
Polyethylenglykol(22)stearat, Polyethylenglykol(23)stearat,
Polyethylenglykol(24)stearat, Polyethylenglykol(25)stearat,
Polyethylenglykol(12)isostearat, Polyethylenglykol(13)isostearat,
Polyethylenglykol(14)isostearat, Polyethylenglykol(15)isostearat,
Polyethylenglykol(16)isostearat, Polyethylenglykol(17)isostearat,
Polyethylenglykol(18)isostearat, Polyethylenglykol(19)isostearat,
Polyethylenglykol(20)isostearat, Polyethylenglykol(21)isostearat,
Polyethylenglykol(22)isostearat, Polyethylenglykol(23)isostearat,
Polyethylenglykol(24)isostearat, Polyethylenglykol(25)isostearat,
Polyethylenglykol(12)oleat, Polyethylenglykol(13)oleat, Polyethylenglykol(14)oleat,
Polyethylenglykol(15)oleat, Polyethylenglykol(16)oleat, Polyethylenglykol(17)oleat,
Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat,
Polyethylenglykol(6)glycerylcaprat/caprinat, Polyethylenglykol(20)glyceryloleat, Polyethylenglykol(20)glycerylisostearat und
Polyethylenglykol(18)glyceryloleat/cocoat zu wählen.

Unter den Sorbitanestem eignen sich besonders
Polyethylenglykol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat.

Besonders vorteilhafte Coemulgatoren sind Glycerylmonostearat, Glycerylmonooleat, Diglycerylmonostearat, Glycerylisostearat, Polyglyceryl-3-oleat, Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglycol(2)stearylether (Steareth-2), Alkylmethiconcopolyole und Alkyl-Dimethiconcopolyole, insbesondere Cetyldimethiconcopolyol, Laurylmethiconcopolyol.

Die erfindungsgemäßen Formulierungen können einen oder mehrere der Emulgatoren, Co-Emulgatoren oder Solubilisatoren in Mengen von 0,1 bis 20,0 Gew.-%, vorzugsweise 1,0 bis 15,0 Gew.-% und besonders bevorzugt 3,0 bis 10,0 Gew.%, bezogen auf die fertigen Formulierungen, enthalten.

Als Elektrolyt zum Einsatz können kommen anorganische Salze, bevorzugt Ammonium- oder Metallsalze, besonders bevorzugt von Halogeniden, beispielsweise CaCl₂, MgCl₂, LiCl, KCI und NaCl, Carbonaten, Hydrogencarbonaten, Phosphaten, Sulfaten, Nitraten, insbesondere bevorzugt Natriumchlorid, und/oder organische Salze, bevorzugt Ammonium- oder Metallsalze, besonders bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure oder Galacturonsäure.
Hierzu zählen auch Aluminiumsalze, bevorzugt Aluminiumchlorohydrat oder Aluminium-Zirkonium-Komplexsalze.

Als Elektrolyt können die erfindungsgemäßen Formulierungen auch Mischungen verschiedener Salze enthalten. Der Gehalt an dem einen oder den mehreren Elektrolyten, bezogen auf die gesamte erfindungsgemäße Formulierung ist vorzugsweise von 0,1 bis 20,0 Gew.-%, besonders bevorzugt von 0,2 bis 10,0 Gew.-% und insbesondere bevorzugt von 0,5 bis 5,0 Gew.-%.

An Hydroxysäuren können die erfindungsgemäßen Formulierungen vorzugsweise Milchsäure, Glykolsäure, Salicylsäure, Zitronensäure oder Polyglykoldisäuren in freier oder teilweiser Neutralisation enthalten. Weiterhin können erfindungsgemäße Formulierungen enthaltend Vitamin C oder Vitamin C-Derivate, Dihydroxyaceton oder Skin-whitening Actives wie Arbutin oder Glycyrrhetinsäure und deren Salze stabilisiert werden. Der Gehalt an einer oder mehreren dieser soeben genannten Substanzen, bezogen auf die gesamte erfindungsgemäße Formulierung, ist vorzugsweise von 0,1 bis 20,0 Gew.-%, besonders bevorzugt von 0,2 bis 10,0 Gew.-% und insbesondere bevorzugt von 0,5 bis 5,0 Gew.-%.

Zu den erfindungsgemäßen Copolymeren können als zusätzliche Stabilisatoren Metallsalze von Fettsäuren, wie z. B. Magnesium-, Aluminium- und/oder Zinkstearat eingesetzt werden, bevorzugt in Mengen von 0,1 bis 10,0 Gew.-%, vorzugsweise von 0,5 bis 8,0 Gew.% und besonders bevorzugt von 1,0 bis 5,0 Gew.-%, bezogen auf die fertigen Formulierungen.

Als kationische Polymere eignen sich die unter der INCI-Bezeichnung "Polyquaternium" bekannten, insbesondere Polyquatemium-31, Polyquaternium-16, Polyquaternium-24, Polyquatemium-7, Polyquatemium-22, Polyquaternium-39, Polyquatemium-28, Polyquaternium-2, Polyquatemium-10, Polyquaternium-11, sowie Polyquatemium 37&mineral oil&PPG trideceth (Salcare SC95), PVP-dimethylaminoethylmethacrylat-Copolymer, Guarhydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quatemierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Die erfindungsgemäßen Formulierungen können einen oder mehrere der oben genannten kationischen Polymere in Mengen von 0,1 bis 5,0 Gew.-%, vorzugsweise von 0,2 bis 3,0 Gew.-% und besonders bevorzugt von 0,5 bis 2,0 Gew.-%, bezogen auf die fertigen Formulierungen, enthalten.

Des Weiteren können die erfindungsgemäßen Formulierungen Filmbildner enthalten, die je nach Anwendungszweck ausgewählt sind aus Salzen der Phenylbenzimidazolsulfonsäure, wasserlöslichen Polyurethanen, beispielsweise C₁₀-Polycarbamylpolyglycerylester,Polyvinylalkohol, Polyvinylpyrrolidoncopolymeren, beispielsweise VinylpyrrolidonNinylacetatcopolymer, wasserlöslichen Acrylsäurepolymeren/Copolymeren bzw. deren Estern oder Salzen, beispielsweise Partialestercopolymere der Acryl/Methacrylsäure, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich.

Die erfindungsgemäßen Formulierungen können einen oder mehrere Filmbildner in Mengen von 0,1 bis 10,0 Gew.%, vorzugsweise von 0,2 bis 5,0 Gew.% und besonders bevorzugt von 0,5 bis 3,0 Gew.-%, bezogen auf die fertigen Formulierungen, enthalten.

Die gewünschte Viskosität der Formulierungen kann durch Zugabe von weiteren Verdickern und Gelierungsmittel eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/ VP-Copolymer Verwendung finden.

In einer weiteren bevorzugten Ausführungsform der Erfindung enthalten die erfindungsgemäßen Formulierungen 0,01 bis 20,0 Gew.-%, besonders bevorzugt 0,1 bis 10,0 Gew.-%, insbesondere bevorzugt 0,2 bis 3,0 Gew.-% und ganz besonders bevorzugt 0,4 bis 2,0 Gew.% an Verdickern bzw. Geliermitteln, bezogen auf die fertigen erfindungsgemäßen Formulierungen.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.% und insbesondere bevorzugt von 0,5 bis 3,0 Gew.-% eingesetzt werden, bezogen auf die fertigen erfindungsgemäßen Formulierungen.

An antimikrobiellen Wirkstoffen können Cetyltrimethylammoniumchlorid, Cetylpyridiniumchlorid, Benzethoniumchlorid,
Diisobutylethoxyethyldimethylbenzylammoniumchlorid,
Natriumaluminiumchlorohydroxylactat, Triethylcitrat,
Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Famesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox^{®}, lodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylhamstoff, Dimethyloldimethylhydantoin (DMDMH), Na-Salz von Hydroxymethylglycin und Kombinationen dieser Wirksubstanzen zum Einsatz kommen.

Die erfindungsgemäßen Formulierungen enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen erfindungsgemäßen Formulierungen.

Die erfindungsgemäßen Formulierungen können des Weiteren biogene Wirkstoffe, ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol^{®}, Allantoin, Phytantriol^{®}, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Die erfindungsgemäßen Formulierungen können biogene Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.%, bezogen auf die fertigen Formulierungen, enthalten.

Die erfindungsgemäßen Formulierungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate bevorzugt in Mengen von 0 bis 50,0 Gew.-%, besonders bevorzugt in Mengen von 0,01 bis 10,0 Gew.% und insbesondere bevorzugt in Mengen von 0,1 bis 10,0 Gew.-% enthalten. Als deodorierende Stoffe bevorzugt sind Allantoin und Bisabolol. Diese werden vorzugsweise in Mengen von 0,0001 bis 10,0 Gew.-% eingesetzt.

Die erfindungsgemäßen Formulierungen können als Pigmente/Mikropigmente sowie als anorganische Sonnenschutzfilter mikrofeines Titandioxid, Glimmer-Titanoxid, Eisenoxide, Glimmer-Eisenoxid, Zinkoxid, Siliciumoxide, Ultramarinblau, Chromoxide enthalten.

Die erfindungsgemäßen Formulierungen können einen oder mehrere organische Sonnenschutzfilter enthalten, vorzugsweise ausgewählt aus 4-Aminobenzoesäure, 3-(4'-Trimethylammonium)-benzyliden-boran-2-on-methylsulfat, Camphor Benzalkonium Methosulfat, 3,3,5-Trimethyl-cyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure) und ihre Salze, 1-(4-tert.-Butylphenyl)-3-(4-methoxyphenyl)propan-1,3-dion, 3-(4'-Sulfo)-benzyliden-boman-2-on und seine Salze, 2-Cyan-3,3-diphenyl-acrylsäure-(2-ethylhexylester), Polymere von N-[2(und 4)-(2-oxoborn-3-ylidenmethyl)benzyl]-acrylamid, 4-Methoxy-zimtsäure-2-ethyl-hexylester, ethoxyliertes Ethyl-4-amino-benzoat, 4-Methoxy-zimtsäure-isoamylester, 2,4,6-Tris-[p-(2-ethylhexyloxycarbonyl)anilino]-1,3,5-triazin, 2-(2H-benzotriazol-2-yl)-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-amino-carbonyl)phenylamino]-1,3,5-triazin-2,4-yl)diimino]bis-(benzoesäure-2-ethylhexylester), Benzophenon-3, Benzophenon-4 (Säure), 3-(4'-Methylbenzyliden)-D,L-Campher, 3-Benzyliden-Campher, Salicylsäure-2-ethylhexylester, 4-Dimethylaminobenzoesäure-2-ethylhexylester, Hydroxy-4-methoxy-benzophenon-5-sulfonsäure (Sulfisobenzonum) und das Natriumsalz, 4-Isopropylbenzylsalicylat, N,N,N-Trimethyl-4-(2-oxoborn-3-ylidenemethyl) anilium methyl sulphate, Homosalate (INN), Oxybenzone (INN), 2-Phenylbenzimidazole-5-sulfonsäure und ihre Natrium-, Kalium-, und Triethanolaminsalze, Octylmethoxyzimtsäure, Isopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometrizole Trisiloxane) benzoesäure, 4,4-((6-(((1,1-dimethylethyl)amino)carbonyl)phenyl)amino)-1,3,5-triazine-2,4-diyl)diimino)bis,bis(2-ethylhexyl)ester) benzoesäure, 4,4-((6-(((1,1-dimethylethyl)amino)-carbonyl)phenyl)amino)-1,3,5-triazine-2,4-diyl)dümino)bis,bis(2-ethylhexyl)ester), 3-(4'-Methylbenzyliden)-D,L-campher (4-Methylbenzyliden Camphor), Benzyliden-camphor-sulfonsäure, Octocrylen, Polyacrylamidomethyl-Benzyliden-Camphor, 2-Ethylhexyl salicylat (Octyl Salicylat), 4-Dimethyl-aminobenzoesäureethyl-2-hexylester (octyl dimethyl PABA), PEG-25 PABA, 2-Hydroxy-4-methoxybenzo-phenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1 H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat, Di-p-methoxyzimtsäure, p-Amino-benzoesäure und deren Ester, 4-tert-Butyl-4'-methoxydibenzoylmethan, 4-(2-β-Glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-Dihydroxy-4,4'-dimethoxy-5,5'-disulfobenzophenon, Dihydroxybenzophenon, 1,3,4-Dimethoxyphenyl-4,4-dimethyl-1,3-pentanedion, 2-Ethylhexyl-dimethoxybenzyliden-dioxoimidazolidinpropionat, Methylen-Bis-Benztriazolyl Tetramethylbutylphenol, Phenyldibenzimidazoltetrasulfonat, Bis-Ethylhexyloxyphenol-Methoxyphenol-Triazin, Tetrahydroxybenzophenone, Terephthalylidendicampher-sulfonsäure, 2,4,6-tris[4,2-Ethylhexyloxycarbonyl)anilino]-1,3,5-triazin, Methyl-bis(trimethylsiloxy)silyl-isopentyl trimethoxy-zimtsäure, Amyl-p-dimethylaminobenzoat, Amyl-p-dimethylamino benzoat, 2-Ethylhexyl-p-dimethylaminobenzoat, Isopropyl-p-methoxyzimtsäurel Diisopropylzimtsäureester, 2-Ethylhexyl-p-methoxyzimtsäure, 2-Hydroxy-4-methoxy benzophenon, 2-Hydroxy-4-methoxybenzophenon-5-sulfsäure und das Trihydrat, sowie 2-Hydroxy-4-methoxybenzophenon-5-sulfonat Natriumsalz und Phenyl-benzimidazol-sulfonsäure.

Die Menge der vorgenannten Sonnenschutzfilter (eine oder mehrere Verbindungen) in den erfindungsgemäßen Formulierungen beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der fertigen Formulierung.

Die erfindungsgemäßen Formulierungen können einen oder mehrere Antioxidantien enthalten, vorzugsweise ausgewählt aus Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (z. B. Ester, Ether, Peptide, Lipide, Nukleotide, Nukleoside und Salze), sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin E-acetat), Vitamin A und Derivaten (Vitamin A-palmitat) sowie Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z. B. Selenmethionin), Stilbenen und deren Derivaten (z. B. Stilbenoxid, trans-Stilbenoxid), Superoxid-Dismutase und den erfindungsgemäß geeigneten Derivaten (Salzen, Estern, Ethern, Zuckern, Nukleotiden, Nukleosiden, Peptiden und Lipiden) dieser genannten Stoffe.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den erfindungsgemäßen Formulierungen beträgt vorzugsweise von 0,001 bis 30,0 Gew.-%, besonders bevorzugt von 0,05 bis 20,0 Gew.% und insbesondere bevorzugt von 1,0 bis 10,0 Gew.-%, bezogen auf das Gesamtgewicht der Formulierung.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxysäuren, Panthenol und dessen Derivaten, beispielsweise D-Panthenol (R-2,4-Dihydroxy-N-(3-hydroxypropyl)-3,3-dimethylbutamid), D,L-Panthenol, Calciumpantothenat, Panthetin, Pantothein, Panthenylethylether, Isopropylpalmitat, Glycerin und/oder Sorbitol eingesetzt werden, bevorzugt in Mengen von 0,1 bis 15,0 Gew.% und besonders bevorzugt von 0,5 bis 5,0 Gew.-%, bezogen auf die fertigen Formulierungen.

Zusätzlich können die erfindungsgemäßen Formulierungen organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45,0 Gew.-% und von Polyethylenglykol mit einer relativen Molekülmasse zwischen 400 und 600 in Mengen von 5,0 bis 25,0 Gew.-% bevorzugt. Weitere geeignete Lösungsmittel sind beispielsweise Triacetin (Glycerintriacetat) und 1-Methoxy-2-propanol.

Die erfindungsgemäßen Formulierungen können ein oder mehrere Substanzen ausgewählt aus Farbmitteln, z. B. Farbstoffe und/oder Pigmente enthalten. Die in den erfindungsgemäßen Formulierungen enthaltenen Farbstoffe und/oder Pigmente, sowohl organische als auch anorganische Farbstoffe und Pigmente, sind aus der entsprechenden Positivliste der Kosmetikverordnung bzw. der EG-Liste kosmetischer Färbemittel ausgewählt.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-brezol-sulfosäure | 13015 | gelb |
| 2,4-Dihydroxy-azobenzol-4'-sulfosäure | 14270 | orange |
| 2-(2,4-Dimethylphenylazo-5-sulfosäurse)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| . 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1'-naphthylazo]-1-hydroxy-7-aminoaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenrylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| .4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| . Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium-, und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z. B. Paprikaextrakte, β-Carotin und Cochenille.

Vorteilhaft eingesetzt werden auch Perlglanzpigmente, z. B. Fischsilber (Guanin/ Hypoxanthin-Mischkristalle aus Fischschuppen) und Perimutt (vermahlene Muschelschalen), monokristalline Periglanzpigmente wie z. B. Bismuthoxychlorid (BiOCI), Schicht-Substrat Pigmente, z. B. Glimmer/Metalloxid, silberweiße Perlglanzpigmente aus TiO₂, Interferenpigmente (TiO₂, unterschiedliche Schichtdicke), Farbglanzpigmente (Fe₂O₃) und Kombinationspigmente (TiO₂/Fe₂O₃, TiO₂/Cr₂O₃, TiO₂/Berliner Blau, TiO₂/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z. B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z. B. Bismutoxychlorid (BiOCI), sowie die Oxide von beispielsweise Titan, insbesondere die TiO₂-Modifikationen Anatas und Rutil, Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z. B. von 2-50 µm, 5-25 µm, 5-40 µm, 5-60 µm, 5-95 µm, 5 - 100 µm, 10-60 µm, 10 - 100 µm, 10 - 125 µm, 20 - 100 µm, 20-150 µm, sowie < 15 µm. Eine breitere Teilchengrößenverteilung z. B. von 20-150 µm, ruft glitzernde Effekte hervor, während eine engere Teilchengrößenverteilung von < 15 µm für eine gleichmäßige seidige Erscheinung sorgt.

Die erfindungsgemäßen Formulierungen enthalten Effektpigmente vorzugsweise in Mengen von 0,1 bis 20,0 Gew.-%, besonders bevorzugt von 0,5 bis 10,0 Gew.% und insbesondere bevorzugt von 1,0 bis 5,0 Gew.-%, jeweils bezogen auf das Gesamtgewicht der Formulierungen.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykol-distearate mit durchschnittlich 3 Glykoleinheiten.

Sofern die erfindungsgemäßen Formulierungen perlglanzgebende Verbindungen enthalten, sind diese bevorzugt in einer Menge von 0,1 bis 15,0 Gew.-% und besonders bevorzugt in einer Menge von 1,0 bis 10,0 Gew.-% in den erfindungsgemäßen Formulierungen enthalten.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Säuren oder Laugen zur pH-Wert Einstellung werden vorzugsweise Mineralsäuren, insbesondere HCl, anorganische Basen, insbesondere NaOH oder KOH, und organische Säuren, insbesondere Zitronensäure, verwendet.

Die Formulierungen haben pH-Werte von vorzugsweise 2 bis 10, besonders bevorzugt von 3 bis 9, insbesondere bevorzugt von 4,5 bis 8 und außerordentlich bevorzugt von 5,5 bis 7,5.

Die nachfolgenden Beispiele und Anwendungen sollen die Erfindung näher erläutern, ohne sie jedoch darauf zu beschränken. Bei allen Prozentangaben handelt es sich um Gewichtsprozent (Gew.-%), sofern nicht explizit anders angegeben.

### Beispiele

Die nachfolgende Auflistung zeigt erfindungsgemäße Copolymere, die für die Formulierung der erfindungsgemäßen Mittel besonders vorteilhaft geeignet sind. Die verschiedenen Copolymere sind gemäß den folgenden Herstellverfahren a), b1) und b2) erhältlich.

### Verfahren a):

Diese Polymere sind mittels radikalischer Polymerisation in tert.-Butanol herstellbar. Dabei wurden die Monomere in tert.-Butanol vorgelegt, die Reaktionsmischung inertisiert und anschließend die Reaktion nach Anheizen auf 60 °C durch Zugabe des entsprechenden tert.-Butanol löslichen Initiators (bevorzugt Dilauroylperoxid) gestartet. Die Polymere werden nach beendeter Reaktion (2 Stunden) durch Abdestillieren des Lösungsmittels und durch anschließende Vakuumtrocknung isoliert.

### Copolymer Nr. 1

| | |
|---|---|
| 350 g | tert.-Butanol mit 2,5 % Wasser |
| 58,2 g | Stearylacrylat |
| 1,8 g | [2-(Acryloyloxy)-ethyl]trimethylammoniumchlorid (AOETAC) |
| 2,0 g | Dilauroylperoxid (DLP) |

### Durchführung:

350 g tert.-Butanol und 58,2 g Stearylacrylat werden im 1 I Kolben mit Rührer, Temperaturfühler, Rückflusskühler und Einleitungsmöglichkeit für N₂ vorgelegt und der Kolben verschlossen. Dann wird mit N₂ inertisiert und anschließend 1,8 g (2-(Acryloyloxyrethyl]trimethylammoniumchlorid (AOETAC) zugegeben. Bei einer Innentemperatur von 60 °C wird die Reaktion mit 2,0 g Dilauroylperoxid (DLP) gestartet. Das entstehende Polymer bleibt dabei in Lösung. Es wird nachgerührt, danach die Innentemperatur auf Rückflusstemperatur erhöht und ausreichend nachgerührt. Das Produkt wird bei 35 °C im Vakuumtrockenschrank getrocknet.

### Verfahren b):

Diese Polymere sind nach dem Lösungsverfahren in organischen Lösungsmitteln (bevorzugt Toluol, z. B. auch tertiäre Alkohole) herstellbar. Dabei werden die Monomere im Lösungsmittel vorgelegt, die Reaktionsmischung inertisiert und anschließend die Reaktion nach Aufheizen auf 60 °C durch Zugabe von geeigneten Initiatoren oder Initiatorsystemen (bevorzugt Dilauroylperoxid) gestartet. Nach beendeter Reaktion werden die Polymere durch Abdampfen oder Kühlen des Lösungsmittels ausgefällt oder durch Fällen in einem Lösungsmittel, in dem das Polymer unlöslich ist, isoliert und anschließend im Vakuum getrocknet.

### Verfahren b1): Eintopfverfahren

### Copolymer Nr. 276

| | |
|---|---|
| 350 g | tert.-Butanol mit 2,5% Wasser |
| 58,2 g | Stearylacrylat |
| 1,8 g | [2-(Acryloyloxy)-ethyl]trimethylammoniumchlorid (AOETAC) und |
| 6,0 g | Laurylmethacrylat |
| 2,0 g | Dilauroylperoxid (DLP) |

### Durchführung:

350 g tert.-Butanol und 58,2 g Stearylacrylat werden im 1 I Kolben mit Rührer, Temperaturfühler, Rückflusskühler und Einleitungsmöglichkeit für N₂ vorgelegt und der Kolben verschlossen. Dann wird mit N₂ inertisiert und anschließend 1,8 g [2-(Acryloyloxy)-ethyl]trimethylammoniumchlorid (AOETAC) und 6,0 g Laurylmethacrylat zugegeben. Bei einer Innentemperatur von 60 °C wird die Reaktion mit 2,0 g Dilauroylperoxid (DLP) gestartet. Das entstehende Polymer bleibt dabei in Lösung. Es wird nachgerührt und anschließend die Innentemperatur auf Rückflusstemperatur erhöht. Nach einer ausreichenden Nachrührzeit wird die Heizung abgeschaltet und auf Raumtemperatur (RT) abgekühlt. Dabei fällt das Polymer aus. Der Niederschlag wird über eine Filternutsche abgesaugt, das Produkt gewaschen und bei 35 °C im Vakuumtrockenschrank getrocknet.

### Verfahren b2): Ausfällung in kaltem Wasser

### Copolymer Nr. 1

| | |
|---|---|
| 350 g | tert.-Butanol mit 2,5% Wasser |
| 58,2 g | Stearylacrylat |
| 1,8 g | [2-(Acryloyloxy)-ethyl]trimethylammoniumchlorid (AOETAC) |
| 2,0 g | Dilauroylperoxid (DLP) |

### Durchführung:

350 g tert.-Butanol und 58,2 g Stearylacrylat werden im 1 I Kolben mit Rührer, Temperaturfühler, Rückflusskühler und Einleitungsmöglichkeit für N₂ vorgelegt und der Kolben verschlossen. Dann wird mit N₂ inertisiert und anschließend 1,8 g [2-(Acryloyloxy)-ethyl]trimethylammoniumchlorid (AOETAC) zugegeben. Bei einer Innentemperatur von 60 °C wird die Reaktion mit 2,0 g Dilauroylperoxid (DLP) gestartet. Das entstehende Polymer bleibt dabei in Lösung. Es wird nachgerührt, danach die Innentemperatur auf Rückflusstemperatur erhöht und ausreichend nachgerührt. Dann wird der heiße Ansatz mittels einer Pumpe in 700 g eiskaltes Wasser gepumpt. Dabei entsteht ein weißer Niederschlag. Dieser wird kalt über einen Weissband-Rundfilter abgesaugt und bei 35 °C im Vakuumtrockenschrank getrocknet.

In folgender Tabelle A sind Beispiele für erfindungsgemäße Polymere aufgeführt. Dabei sind in der ersten Spalte Monomere der folgenden Strukturformel (Ia1) 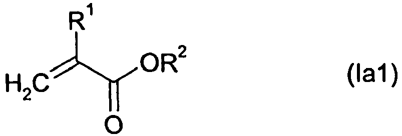 gemäß Komponente a) wiedergegeben.

**Tabelle A Beispiele erfindungsgemäßer Polymere**

| Polymer Nr. | Komponente a) | Komponente b) | Komponente c) | Komponente a) | Komponente a) oder c) | Herstellweg |
|---|---|---|---|---|---|---|
| | Im Folgenden: R² der Formel (Ia1) mit R¹ = H | | | | | |
| 1 | 97 % C₁₈H₃₇ | 3 % AOETAC | AOETAC - | - | - | a |
| 2 | 97 % C₁₈H₃₇ | 2 % AOETAC | 1 % EDDMA | - | - | a |
| 3 | 98 % C₁₆H₃₇ | 1 % AOETAC | 1 % HDDMA | - | - | a |
| 4 | 97 % C₁₈H₃₇ | 2 % AOETAC | 1 % HDDMA | - | - | a |
| 5 | 96 % C₁₈H₃₇ | 3 % AOETAC | 1 % HDDMA | - | - | a |
| 6 | 94 % C₁₈H₃₇ | 5 % AOETAC | 1 % HDDMA | - | - | a |
| 7 | 89 % C₁₈H₃₇ | 10 % AOETAC | 1 % HDDMA | - | - | a |
| 8 | 96,5 % C₁₈H₃₇ | 2 % AOETAC | 1,5 % HDDMA | - | - | a |
| 9 | 96 % C₁₈H₃₇ | 2 % AOETAC | 2 % HDDMA | - | - | a |
| 10 | 95 % C₁₈H₃₇ | 2 % AOETAC | 3 % HDDMA | - | - | a |
| 11 | 93 % C₁₈H₃₇ | 2 % AOETAC | 5 % HDDMA | - | - | a |
| 12 | 97 % C₁₈H₃₇ | 2 % AOETAC | 1 % BDDMA | - | - | a |
| 13 | 97 % C₁₈H₃₇ | 2 % AOETAC | 1 % DDDMA | - | - | a |
| 14 | 97 % C₁₈H₃₇ | 2 % AOETAC | 1 % TMPTA | - | - | a |
| 15 | 97 % C₁₈H₃₇ | 2 % AOETAC | 1 % TMPTMA | - | - | a |
| 16 | 97 % C₁₈H₃₇ | 2 % AOETAC | 1 % MBA | - | - | a |
| 17 | 97 % C₁₈H₃₇ | 2 % AOETAC | 1 % HDDMA/TMPTA | HDDMA/TMPTA - | - | a |
| 18 | 97 % C₁₈H₃₇ | 2 % AOETAC | 1 % AMA | - | - | a |
| 19 | 97 % C₁₈H₃₇ | 2 % AOETAC | 1 % Glycerindiacrylat | Glycerindiacrylat - | - | a |
| 20 | 97 % C₁₈H₃₇ | 2 % AOETAC | 1 % Triallylamin | - | - | a |
| 21 | 97 % C₁₈H₃₇ | 2 % AOETAC | 1 % Triallylcyanurat | - | - | a |
| 22 | 97 % C₁₈H₃₇ | 2 % AOETAC | 1 % PEG-Diacrylat | - | - | a |
| 23 | 93 % C₁₈H₃₇ | 3 % AOETAC | - | 4 % B250MA | - | a |
| 24 | 89 % C₁₈H₃₇ | 3 % AOETAC | - | 8 % B250MA | - | a |
| 25 | 87 % C₁₈H₃₇ | 3 % AOETAC | - | 10 % B250MA | - | a |
| 26 | 85 % C₁₈H₃₇ | 3 % AOETAC | - | 12 % B250MA | - | a |
| 27 | 91 % C₁₈H₃₇ | 3 % AO ETAC | 1 % HDDMA | 5 % B250MA | - | a |
| 28 | 86 % C₁₈H₃₇ | 3 % AOETAC | 1 % HDDMA | 10 % B250MA | - | a |
| 29 | 76,2 % C₁₈H₃₇ | 3 % AOETAC | 1 % HDDMA | 19,8 % B250MA | - | a |
| 30 | 56,4 % C₁₈H₃₇ | 3 % AOETAC | 1 % HDDMA | 39,6 % B250MA | - | a |
| 31 | 82,9 % C₁₈H₃₇ | 2,9% AOETAC | 4,7% HDDMA | 9,5 % B250MA | - | a |
| 32 | 79,1 % C₁₈H₃₇ | 2,7% AOETAC | 9,1 % HDDMA | 9,1 % B250MA | - | a |
| 33 | 78,7 % C₁₈H₃₇ | 2,7% AOETAC | 4,8% HDDMA | 9 % B250MA | 4,8 % Hexadecylacrylat | a |
| 34 | 74,6 % C₁₈H₃₇ | 2,5% AOETAC | 4,8% HDDMA | 8,6 % B250MA | 9,5 % Hexadecylacrylat | a |
| 35 | 66,3 % C₁₈H₃₇ | 2,3% AOETAC | 4,8% HDDMA | 7,6 % B250MA | 19 % Hexadecylacrylat | a |
| 36 | 78,7 % C₁₈H₃₇ | 2,7% AOETAC | 4,8% HDDMA | 9 % B250MA | 4,8 % Octyldodecylacrylat | a |
| 37 | 74,6 % C₁₈H₃₇ | 2,5% AOETAC | 4,8% HDDMA | 8,6 % B250MA | 9,5% Octyldodecylacrylat | a |
| 38 | 66,3 % C₁₈H₃₇ | 2,3% AOETAC | 4,8% HDDMA | 7,6 % B250MA | 19 % Octyldodecylacrylat | a |
| 39 | 78,7 % C₁₈H₃₇ | 2,7% AOETAC | 4,8% HDDMA | 9 % B250MA | 4,8 % Isostearylacrylat | a |
| 40 | 74,6 % C₁₈H₃₇ | 2,5% AOETAC | 4,8% HDDMA | 8,6 % B250MA | 9,5 % Isostearylacrylat | a |
| 41 | 66,3 % C₁₈H₃₇ | 2,3% AOETAC | 4,8% HDDMA | 7,6 % B250MA | 19 % Isostearylacrylat | a |
| 42 | 78,7 % C₁₈H₃₇ | 2,7% AOETAC | 4,8% HDDMA | 9 % B250MA | 4,8 % C₂₆-C₂₈ α-Olefin | a |
| 43 | 74,6 % C₁₈H₃₇ | 2,5% AOETAC | 4,8% HDDMA | 8,6 % B250MA | 9,5 % C₂₆-C₂₈ α-Olefin | a |
| 45 | 84,2 % C₁₈H₃₇ | 1,6% AOETAC | 3,3% DDDMA | 9,3 % B250MA | 1,6 % C₂₈-C₂₈ α-Olefin | a |
| 46 | 82,8 % C₁₈H₃₇ | 4,2% AOETAC | 3,3% DDDMA | 4,7 % B250MA | 5 % C₂₈-C₂₈ α-Olefin | a |
| 47 | 78,4 % C₁₈H₃₇ | 5,2% AOETAC | 5 % DDDMA | 4,7 % B250MA | 6,7 % C₂₆-C₂₈ α-Olefin | a |
| 48 | 78,7 % C₁₈H₃₇ | 2,7% AOETAC | 4,8% DDDMA | 9 % B250MA | 4,8 % C₃₀ α-Olefin | a |
| 49 | 81,3 % C₁₈H₃₇ | 2,8% AOETAC | 1,6% TMPTA | 9,3 % B250MA | 5 % C₂₆-C₂₈ α-Olefin | a |
| 50 | 80 % C₁₈H₃₇ | 2,8% AOETAC | 3,2% TMPTA | 9,2 % B250MA | 4,8 % C₂₆-C₂₆ α-Olefin | a |
| 51 | 81,9 % C₁₈H₃₇ | 1,6% AOETAC | 3,3% TMPTA | 9,9 % B250MA | 3,3 % C₂₆-C₂₈ α-Olefin | a |
| 52 | 84,1 % C₁₈H₃₇ | 1,6% AOETAC | 3,3% TMPTA | 9,4 % B250MA | 1,6 % C₂₆-C₂₈ α-Olefin | a |
| 53 | 88,5 % C₁₈H₃₇ | 1,7% AOETAC | 3,4% TMPTA | 4,7 % B250MA | 1,7 % C₂₆-C₂₆ α-Olefin | a |
| 54 | 81,3 % C₁₈H₃₇ | 1,7% AOETAC | 3,3% TMPTA | 12 % B250MA | 1,7 % C₂₆-C_{28 α}-Olefin | a |
| 55 | 80,7 % C₁₈H₃₇ | 0,8% AOETAC | 3,3% TMPTA | 10,2 % B250MA | 5 % C₂₆-C₂₈ α-Olefin | a |
| 56 | 83,2 % C₁₈H₃₇ | 1,7% AOETAC | 3,3% TMPTA | 8,4 % B250MA | 3,4 % C₂₆-C₂₈ α-Olefin | a |
| 57 | 78,7 % C₁₈H₃₇ | 2,7% AOETAC | 4,8% TMPTA | 9 % B250MA | 4,8 % C₂₆-C₂₈ α-Olefin | a |
| 58 | 82,5 % C₁₈H₃₇ | 0,8% AOETAC | 5 % TMPTA | 8,4 % B250MA | 3,3 % C₂₆-C₂₈ α-Olefin | a |
| 59 | 82,9 % C₁₈H₃₇ | 2,9% AOETAC | 4,7% HDDMA/TMPTA | 9,5 % B250MA | - | a |
| 60 | 78,2 % C₁₈H₃₇ | 3,6% AOETAC | 4,7% HDDMA/TMPTA | 9 % B250MA | 4,7 % C₂₆-C₂₈ α-Olefin | a |
| 61 | 91 % C₁₈H₃₇ | 3 % AOETAC | 1 % HDDMA | 5 % T250MA | - | a |
| 62 | 86,1 % C₁₈H₃₇ | 3 % AOETAC | 1 % HDDMA | 9,9 % T250MA | - | a |
| 63 | 91 % C₁₆H₃₇ | 3 % AOETAC | 1 % HDDMA | 5 % T080MA | - | a |
| 64 | 86,1 % C₁₈H₃₇ | 3 % AOETAC | 1 % HDDMA | 9,9 % T080MA | | |
| 65 | 91 % C₁₈H₃₇ | 3 % AOETAC | 1 % HDDMA | 5 % LA070MA | - | a |
| 66 | 86,1 % C₁₈H₃₇ | 3 % AOETAC | 1 % HDDMA | 9,9 % LA070MA | | |
| 67 | 91 %C₁₈H₃₇ | 3 % AOETAC | 1 % HDDMA | - | 5 % Butylacrylat | a |
| 68 | 86,1 % C₁₈H₃₇ | 3 % AOETAC | 1 % HDDMA | - | 9,9 % Butylacrylat | a |
| 69 | 86,1 % C₁₈H₃₇ | 3 % AOETAC | 1 % HDDMA | - | 9,9 % Hexadecylacrylat | a |
| 70 | 86,1 % C₁₈H₃₇ | 3 % AOETAC | 1 % HDDMA | - | 9,9 % Octyldodecylacrylat | a |
| 71 | 86,1 % C₁₈H₃₇ | 3 % AOETAC | 1 % HDDMA | - | 9,9 % Isostearylacrylat | a |
| 72 | 86,1 % C₁₈H₃₇ | 3 % AOETAC | 1 % HDDMA | - | 9,9 % C₂₆-C₂₈ α-Olefin | a |
| 73 | 86,1 % C₁₈H₃₇ | 3 % AOETAC | 1 % HDDMA | - | 9,9 % C₃₀ α-Olefin | a |
| 74 | 97 % C₁₈H₃₇ | 3 % MAPTAC | - | - | - | a |
| 75 | 97 % C₁₈H₃₇ | 2 % MAPTAC | 1 % EDDMA | - | - | a |
| 76 | 97 % C₁₈H₃₇ | 2 % MAPTAC | 1 % HDDMA | - | - | a |
| 77 | 97 % C₁₈H₃₇ | 2 % MAPTAC | 1 % BDDMA | - | - | a |
| 78 | 97 % C₁₈H₃₇ | 2 % MAPTAC | 1 % DDDMA | - | - | a |
| 79 | 97 % C₁₈H₃₇ | 2 % MAPTAC | 1 % TMPTA | - | - | a |
| 80 | 97 % C₁₈H₃₇ | 2 % MAPTAC | 1 % TMPTMA | - | - | a |
| 81 | 97 % C₁₈H₃₇ | 2 % MAPTAC | 1 % MBA | - | - | a |
| 82 | 97 % C₁₈H₃₇ | 2 % MAPTAC | 1 % HDDMA/TMPTA | | - | a |
| 83 | 97 % C₁₈H₃₇ | 2 % MAPTAC | 1 % AMA | - | - | a |
| 84 | 97 % C₁₈H₃₇ | 2 % MAPTAC | 1 % Glycerindiacrylat | - | - | a |
| 85 | 97 % C₁₈H₃₇ | 2 % MAPTAC | 1 % Triallylamin | - | - | a |
| 86 | 97 % C₁₈H₃₇ | 2 % MAPTAC | 1 % Triallylcyanurat | - | - | a |
| 87 | 97 % C₁₈H₃₇ | 2 % MAPTAC | 1 % PEG-Diacrylat | - | - | a |
| 88 | 87 % C₁₈H₃₇ | 3 % MAPTAC | - | 10 % B250MA | - | a |
| 89 | 86 % C₁₈H₃₇ | 3 % MAPTAC | 1 % HDDMA | 10 % B250MA | - | a |
| 90 | 78,7 % C₁₈H₃₇ | 2,7% MAPTAC | 4,8% HDDMA | 9 % B250MA | 4,8 % Butylacrylat | a |
| 91 | 78,7 % C₁₈H₃₇ | 2,7% MAPTAC | 4,8% HDDMA | 9 % B250MA | 4,8 % Hexadecylacrylat | a |
| 92 | 78,7 % C₁₈H₃₇ | 2,7% MAPTAC | 4,8% HDDMA | 9 % B250MA | 4,8 % Octyldodecylacrylat | a |
| 93 | 78,7 % C₁₈H₃₇ | 2,7% MAPTAC | 4,8% HDDMA | 9 % B250MA | 4,8 % Isostearylacrylat | a |
| 94 | 78,7 % C₁₈H₃₇ | 2,7% MAPTAC | 4,8% HDDMA | 9 % B250MA | 4,8 % C₂₆-C₂₈ α-Olefin | a |
| 95 | 78,7 % C₁₈H₃₇ | 2,7% MAPTAC | 4,8% HDDMA | 9 % B250MA | 4,8 % C₃₀ α-Olefin | a |
| 96 | 78,7 % C₁₈H₃₇ | 2,7% MAPTAC | 4,8% DDDMA | 9 % B250MA | 4,8 % C₂₆-C₂₈ α-Olefin | a |
| 97 | 78,7 % C₁₈H₃₇ | 2,7% MAPTAC | 4,8% DDDMA | 9 % B250MA | 4,8 % C₃₀ α-Olefin | a |
| 98 | 78,7 % C₁₈H₃₇ | 2,7% MAPTAC | 4,8% TMPTA | 9 % B250MA | 4,8 % C₂₆-C₂₈ α-Olefin | a |
| 99 | 78,7 % C₁₈H₃₇ | 2,7% MAPTAC | 4,8% TMPTA | 9 % B250MA | 4,8 % C₃₀ α-Olefin | a |
| 100 | 78,7 % C₁₈H₃₇ | 3 % MAPTAC | 1 % HDDMA/TMPTA | 10 % B250MA | - | a |
| 101 | 78,7 % C₁₈H₃₇ | 2,7% MAPTAC | 4,8% HDDMA/TMPTA | 9 % B250MA | 4,8 % C₂₆-C₂₈ α-Olefin | a |
| 102 | 86 % C₁₈H₃₇ | 3 % MAPTAC | 1 % HDDMA | 10 % T250MA | - | a |
| 103 | 86 % C₁₈H₃₇ | 3 % MAPTAC | 1 % HDDMA | 10 % T080MA | - | a |
| 104 | 86 % C₁₈H₃₇ | 3 % MAPTAC | 1 % HDDMA | 10 % LA070MA | - | a |
| 105 | 86,1 % C₁₈H₃₇ | 3 % MAPTAC | 1 % HDDMA | - | 9,9 % Butylacrylat | a |
| 106 | 86,1 % C₁₈H₃₇ | 3 % MAPTAC | 1 % HDDMA | - | 9,9 % Hexadecylacrylat | a |
| 107 | 86,1 % C₁₈H₃₇ | 3 % MAPTAC | 1 % HDDMA | - | 9,9 % Octyldodecylacrylat | a |
| 108 | 86,1 % C₁₈H₃₇ | 3 % MAPTAC | 1 % HDDMA | - | 9,9 % Isostearylacrylat | a |
| 109 | 86,1 % C₁₈H₃₇ | 3 % MAPTAC | 1 % HDDMA | - | 9,9 % C₂₆-C₂₈ α-Olefin | a |
| 110 | 86,1 % C₁₈H₃₇ | 3 % MAPTAC | 1 % HDDMA | - | 9,9 % C₃₀ α-Olefin | a |
| 111 | 78,2 % C₁₈H₃₇ | 3,6% AOETAC/ MAPTAC | 4,7% HDDMA/TMPTA | 9 % B250MA | 4,7 % C₂₆-C₂₈ α-Olefin | a |
| 112 | 97 % C₁₈H₃₇ | 3 % DADMAC | - | - | - | a |
| 113 | 97 %C₁₈H₃₇ | 2 % DADMAC | 1 % EDDMA | - | - | a |
| 114 | 97 % C₁₈H₃₇ | 2 % DADMAC | 1 % HDDMA | - | - | a |
| 115 | 97 % C₁₈H₃₇ | 2 % DADMAC | 1 % BDDMA | - | | a |
| 116 | 97 % C₁₈H₃₇ | 2 % DADMAC | 1 % DDDMA | - | - | a |
| 117 | 97 % C₁₈H₃₇ | 2 % DADMAC | 1 % TMPTA | - | - | a |
| 118 | 86 % C₁₈H₃₇ | 3 % DADMAC | 1 % HDDMA | 10 % B250MA | - | a |
| 119 | 78,7 % C₁₈H₃₇ | 2,7% DADMAC | 4,8% HDDMA | 9 % B250MA | 4,8 % C₂₆-C₂₈ α-Olefin | a |
| 120 | % C₁₈H₃₇ | 2,7% DADMAC | 4,8% HDDMA | 9 % B250MA | 4,8 % C₃₀ α-Olefin | a |
| | Im Folgenden: R² der Formel (la1) mit R¹ = Methyl | | | | | |
| 121 | 97 % C₁₂H₂₅ | 3 % AOETAC | - | - | - | a |
| 122 | 98 % C₁₂H₂₅ | 1 % AOETAC | 1 % HDDMA | - | - | a |
| 123 | 97 % C₁₂H₂₅ | 2 % AOETAC | 1 % HDDMA | - | - | a |
| 124 | 96 % C₁₂H₂₅ | 3 % AOETAC | 1 % HDDMA | - | - | a |
| 125 | 94 % C₁₂H₂₅ | 5 % AOETAC | 1 % HDDMA | - | - | a |
| 126 | 89 % C₁₂H₂₅ | 10 % AOETAC | 1 % HDDMA | - | - | a |
| 127 | 97 % C₁₂H₂₅ | 2 % AOETAC | 1 % DDDMA | - | - | a |
| 128 | 97 % C₁₂H₂₅ | 2 % AOETAC | 1 % TMPTA | - | - | a |
| 129 | 97 % C₁₂H₂₅ | 2 % AOETAC | 1 % HDDMA/TMPTA | | - | a |
| 130 | 93 % C₁₂H₂₅ | 3 % AOETAC | - | 4 % B250MA | - | a |
| 131 | 89 % C₁₂H₂₅ | 3 % AOETAC | | 8 % B250MA | - | a |
| 132 | 87 % C₁₂H₂₅ | 3 % AOETAC | - | 10 % B250MA | - | a |
| 133 | 91 % C₁₂H₂₅ | 3 % AOETAC | 1 % HDDMA | 5 % B250MA | - | a |
| 134 | 86 % C₁₂H₂₅ | 3 % AOETAC | 1 % HDDMA | 10 % B250MA | - | a |
| 135 | 82,9 % C₁₂H₂₅ | 2,9% AOETAC | 4,7% HDDMA | 9,5 % B250MA | - | a |
| 136 | 78,7 % C₁₂H₂₅ | 2,7% AOETAC | 4,8% HDDMA | 9 % B250MA | 4,8 % Hexadecylacrylat | a |
| 137 | 74,6 % C₁₂H₂₅ | 2,5% AOETAC | 4,8% HDDMA | 8,6 % B250MA | 9,5 % Hexadecylacrylat | a |
| 138 | 78,7 % C₁₂H₂₅ | 2,7% AOETAC | 4,8% HDDMA | 9 % B250MA | 4,8 % Octyldodecylacrylat | a |
| 139 | 74,6 % C₁₂H₂₅ | 2,5% AOETAC | 4,8% HDDMA | 8,6 % B250MA | 9,5 % Octyldodecylacrylat | a |
| 140 | 78,7 % C₁₂H₂₅ | 2,7% AOETAC | 4,8% HDDMA | 9 % B250MA | 4,8 % Isostearylacrylat | a |
| 141 | 74,6 % C₁₂H₂₅ | 2,5% AOETAC | 4,8% HDDMA | 8,6 % B250MA | 9,5 % Isostearylacrylat | a |
| 142 | 78,7 % C₁₂H₂₅ | 2,7% AOETAC | 4,8% HDDMA | 9 % B250MA | 4,8 % C₂₆-C₂₈ α-Olefin | a |
| 143 | 74,6 % C₁₂H₂₅ | 2,5% AOETAC | 4,8% HDDMA | 8,6 % B250MA | 9,5 % C₂₆-C₂₈ α-Olefin | a |
| 144 | 78,7 % C₁₂H₂₅ | 2,7% AOETAC | 4,8% DDDMA | 9 % B250MA | 4,8 % C₃₀ α-Olefin | a |
| 145 | 80 % C₁₂H₂₅ | 2,8% AOETAC | 3,2% TMPTA | 9,2 % B250MA | 4,8 % C₂₆-C₂₈ α-Olefin | a |
| 146 | 82,9 % C₁₂H₂₅ | 2,9% AOETAC | 4,7% HDDMA/TMPTA | 9,5 % B250MA | - | a |
| 147 | 78,2 % C₁₂H₂₅ | 3,6% AOETAC | 4,7% HDDMA/TMPTA | 9 % B250MA | 4,7 %C₂₆-C₂₈ α-Ofefin | a |
| 148 | 91 % C₁₂H₂₅ | 3 % AOETAC | 1 % HDDMA | 5 % T250MA | - | a |
| 149 | 86,1 % C₁₂H₂₅ | 3 % AOETAC | 1 % HDDMA | 9,9 % T250MA | - | A |
| 150 | 91 % C₁₂H₂₅ | 3 % AOETAC | 1 % HDDMA | 5 % T080MA | - | A |
| 151 | 86,1 % C₁₂H₂₅ | 3 % AOETAC | 1 % HDDMA | 9,9 % T080MA | | |
| 152 | 91 % C₁₂H₂₅ | 3 % AOETAC | 1 % HDDMA | 5 % LA070MA | - | A |
| 153 | 86,1 % C₁₂H₂₅ | 3 % AOETAC | 1 % HDDMA | 9,9 % LA070MA | | |
| 154 | 91 % C₁₂H₂₅ | 3 % AOETAC | 1 % HDDMA | - | 5 % Butylacrylat | a |
| 155 | 86,1 % C₁₂H₂₅ | 3 % AOETAC | 1 % HDDMA | - | 9,9 % Butylacrylat | a |
| 156 | 86,1 % C₁₂H₂₅ | 3 % AOETAC | 1 % HDDMA | - | 9,9 % Hexadecylacrylat | a |
| 157 | 86,1% C₁₂H₂₅ | 3 % AOETAC | 1 % HDDMA | - | 9,9 % Octyldodecylacrylat | a |
| 158 | 86,1 % C₁₂H₂₅ | 3 % AOETAC | 1 % HDDMA | - | 9,9 % Isostearylacrylat | a |
| 159 | 86,1 % C₁₂H₂₅ | 3 % AOETAC | 1 % HDDMA | - | 9,9 % C₂₆-C₂₈ α-Olefin | a |
| 160 | 86,1 % C₁₂H₂₅ | 3 % AOETAC | 1 % HDDMA | - | 9,9 % C₃₀ α-Olefin | a |
| 161 | 97 % C₁₂H₂₅ | 3 % MAPTAC | - | - | - | a |
| 162 | 97 % C₁₂H₂₅ | 2 % MAPTAC | 1 % HDDMA | - | - | a |
| 163 | 97 % C₁₂H₂₅ | 2 % MAPTAC | 1 % DDDMA | - | - | a |
| 164 | 97 % C₁₂H₂₅ | 2 % MAPTAC | 1 % TMPTA | - | - | a |
| 165 | 97 % C₁₂H₂₅ | 2 % MAPTAC | 1 % HDDMA/TMPTA | - | - | a |
| 166 | 87 % C₁₂H₂₅ | 3 % MAPTAC | - | 10 % B250MA | - | a |
| 167 | 86 % C₁₂H₂₅ | 3 % MAPTAC | 1 % HDDMA | 10 % B250MA | - | a |
| 168 | 78,7 % C₁₂H₂₅ | 2,7% MAPTAC | 4,8% HDDMA | 9 % B250MA | 4,8 % Butylacrylat | a |
| 169 | 78,7 % C₁₂H₂₅ | 2,7% MAPTAC | 4,8% HDDMA | 9 % B250MA | 4,8 % Hexadecylacrylat | a |
| 170 | 78,7 % C₁₂H₂₅ | 2,7% MAPTAC | 4,8% HDDMA | 9 % B250MA | 4,8 % Octyldodecylacrylat | a |
| 171 | 78,7 % C₁₂H₂₅ | 2,7% MAPTAC | 4,8% HDDMA | 9 % B250MA | 4,8 % Isostearylacrylat | a |
| 172 | 78,7 % C₁₂H₂₅ | 2,7% MAPTAC | 4,8% HDDMA | 9 % B250MA | 4,8 % C₂₆-C₂₈ α-Olefin | a |
| 173 | 78,7 % C₁₂H₂₅ | 2,7% MAPTAC | 4,8% HDDMA | 9 % B250MA | 4,8 % C₃₀ α-Olefin | a |
| 174 | 78,7 % C₁₂H₂₅ | 2,7% MAPTAC | 4,8% DDDMA | 9 % B250MA | 4,8 % C₂₆-C₂₈ α-Olefin | a |
| 175 | 78,7 % C₁₂H₂₅ | 2,7% MAPTAC | 4,8% DDDMA | 9 % B250MA | 4,8 % C₃₀ α-Olefin | a |
| 176 | 78,7 % C₁₂H₂₅ | 2,7% MAPTAC | 4,8% TMPTA | 9 % B250MA | 4,8 % C₂₆-C₂₈ α-Olefin | a |
| 177 | 78,7 % C₁₂H₂₅ | 2,7% MAPTAC | 4,8% TMPTA | 9 % B250MA | 4,8 % C₃₀ α-Olefin | a |
| 178 | 78,7 % C₁₂H₂₅ | 3 % MAPTAC | 1 % HDDMA/TMPTA | 10 % B250MA | - | a |
| 179 | 78,7 % C₁₂H₂₅ | 2,7% MAPTAC | 4,8% HDDMA/TMPTA | 9 % B250MA | 4,8 % C₂₆-C₂₈ α-Olefin | a |
| 180 | 86 % C₁₂H₂₅ | 3 % MAPTAC | 1 % HDDMA | 10 % T250MA | - | a |
| 181 | 86 % C₁₂H₂₅ | 3 % MAPTAC | 1 % HDDMA | 10 % T080MA | - | a |
| 182 | 86 % C₁₂H₂₅ | 3 % MAPTAC | 1 % HDDMA | 10 % LA070MA | - | a |
| 183 | 86,1 % C₁₂H₂₅ | 3 % MAPTAC | 1 % HDDMA | - | 9,9 % Butylacrylat | a |
| 184 | 86,1 % C₁₂H₂₅ | 3 % MAPTAC | 1 % HDDMA | - | 9,9 % Hexadecylacrylat | a |
| 185 | 86,1 % C₁₂H₂₅ | 3 % MAPTAC | 1 % HDDMA | - | 9,9 % Octyldodecylacrylat | a |
| 186 | 86,1 % C₁₂H₂₅ | 3 % MAPTAC | 1 % HDDMA | - | 9,9 % Isostearylacrylat | a |
| 187 | 86,1 % C₁₂H₂₅ | 3 % MAPTAC | 1 % HDDMA | - | 9,9 % C₂₆-C₂₈ α-Olefin | a |
| 188 | 86,1 % C₁₂H₂₅ | 3 % MAPTAC | 1 % HDDMA | - | 9,9 % C₃₀ α-Olefin | a |
| 189 | 78,2 % C₁₂H₂₅ | 3,6% AOETAC/ MAPTAC | 4,7% HDDMA/TMPTA | 9 % B250MA | 4,7 % C₂₆-C₂₈ α-Olefin | a |
| 190 | 97 % C₁₂H₂₅ | 3 % DADMAC | - | - | - | a |
| 191 | 97 % C₁₂H₂₅ | 2 % DADMAC | 1 % HDDMA | - | - | a |
| 192 | 97 % C₁₂H₂₅ | 2 % DADMAC | 1 % DDDMA | - | - | a |
| 193 | 97 % C₁₂H₂₅ | 2 % DADMAC | 1 % TMPTA | - | - | a |
| 194 | 86 % C₁₂H₂₅ | 3 % DADMAC | 1 % HDDMA | 10 % B250MA | - | a |
| 195 | 78,7 % C₁₂H₂₅ | 2,7% DADMAC | 4,8% HDDMA | 9 % B250MA | 4,8 % C₂₆-C₂₈ α-Olefin | a |
| 196 | 78,7 % C₁₂H₂₅ | 2,7% DADMAC | 4,8% HDDMA | 9 % B250MA | 4,8 % C₃₀ α-Olefin | a |
| | Im Folgenden: R² der Formel (la1) mit R¹ = H | | | | | |
| 197 | 97 % C₂₂H₄₅ | 3 % AOETAC | - | - | - | a |
| 198 | 98 % C₂₂H₄₅ | 1 % AOETAC | 1 % HDDMA | - | - | a |
| 199 | 97 % C₂₂H₄₅ | 2 % AOETAC | 1 % HDDMA | - | - | a |
| 200 | 96 % C₂₂H₄₅ | 3 % AOETAC | 1 % HDDMA | - | - | a |
| 201 | 94 % C₂₂H₄₅ | 5. % AOETAC | 1 % HDDMA | - | - | a |
| 202 | 89 % C₂₂H₄₅ | 10 % AOETAC | 1 % HDDMA | - | - | a |
| 203 | 97 % C₂₂H₄₅ | 2 % AOETAC | 1 % DDDMA | - | - | a |
| 204 | 97 % C₂₂H₄₅ | 2 % AOETAC | 1 % TMPTA | - | - | a |
| 205 | 97 % C₂₂H₄₅ | 2 % AOETAC | 1 % HDDMA/TMPTA | - | - | a |
| 206 | 93 % C₂₂H₄₅ | 3 % AOETAC | - | 4 % B250MA | - | a |
| 207 | 89 % C₂₂H₄₅ | 3 % AOETAC | - | 8 % B250MA | - | a |
| 208 | 87 % C₂₂H₄₅ | 3 % AOETAC | - | 10 % B250MA | - | a |
| 209 | 91 % C₂₂H₄₅ | 3 % AOETAC | 1 % HDDMA | 5 % B250MA | - | a |
| 210 | 86 % C₂₂H₄₅ | 3 % AOETAC | 1 % HDDMA | 10 % B250MA | | a |
| 211 | 82,9 % C₂₂H₄₅ | 2,9% AOETAC | 4,7% HDDMA | 9,5 % B250MA | - | a |
| 212 | 78,7 % C₂₂H₄₅ | 2,7% AOETAC | 4,8% HDDMA | 9 % B250MA | 4,8 % Hexadecylacrylat | a |
| 213 | 74,6 % C₂₂H₄₅ | 2,5% AOETAC | 4,8% HDDMA | 8,6 % B250MA | 9,5 % Hexadecylacrylat | a |
| 214 | 78,7 % C₂₂H₄₅ | 2,7% AOETAC | 4,8% HDDMA | 9 % B250MA | 4,8 % Octyldodecylacrylat | a |
| 215 | 74,6 % C₂₂H₄₅ | 2,5% AOETAC | 4,8% HDDMA | 8,6 % B250MA | 9,5 % Octyldodecylacrylat | a |
| 216 | 78,7 % C₂₂H₄₅ | 2,7% AOETAC | 4,8% HDDMA | 9 % B250MA | 4,8 % Isostearylacrylat | a |
| 217 | 74,6 % C₂₂H₄₅ | 2,5% AOETAC | 4,8% HDDMA | 8,6 % B250MA | 9,5 % Isostearylacrylat | a |
| 218 | 78,7 % C₂₂H₄₅ | 2,7% AOETAC | 4,8% HDDMA | 9 % B250MA | 4,8 % C₂₆-C₂₈ α-Olefin | a |
| 219 | 74,6 % C₂₂H₄₅ | 2,5% AOETAC | 4,8% HDDMA | 8,6 % B250MA | 9,5 % C₂₆-C₂₈ α-Olefin | a |
| 220 | 78,7 % C₂₂H₄₅ | 2,7% AOETAC | 4,8% DDDMA | 9 % B250MA | 4,8 % C₃₀ α-Olefin | a |
| 221 | 80 % C₂₂H₄₅ | 2,8% AOETAC | 3,2% TMPTA | 9,2 % B250MA | 4,8 % C₂₆-C₂₈ α-Olefin | a |
| 222 | 82,9 % C₂₂H₄₅ | 2,9% AOETAC | 4,7% HDDMA/TMPTA | 9,5 % B250MA | - | a |
| 223 | 78,2 % C₂₂H₄₅ | 3,6% AOETAC | 4,7% HDDMAITMPTA | 9 % B250MA | 4,7 % C₂₆-C₂₈ α-Olefin | a |
| 224 | 91 % C₂₂H₄₅ | 3 % AOETAC | 1 % HDDMA | 5 % T250MA | - | a |
| 225 | 86,1 % C₂₂H₄₅ | 3 % AOETAC | 1 % HDDMA | 9,9 % T250MA | - | a |
| 226 | 91 % C₂₂H₄₅ | 3 % AOETAC | 1 % HDDMA | 5 % T080MA | - | a |
| 227 | 86,1 % C₂₂H₄₅ | 3 % AOETAC | 1 % HDDMA | 9,9 % T080MA | | |
| 228 | 91 % C₂₂H₄₅ | 3 % AOETAC | 1 % HDDMA | 5 % LA070MA | - | a |
| 229 | 86,1 % C₂₂H₄₅ | 3 % AOETAC | 1 % HDDMA | 9,9 % LA070MA | | |
| 230 | 91 % C₂₂H₄₅ | 3 %AOETAC | 1 % HDDMA | - | 5 % Butylacrylat | a |
| 231 | 86,1 % C₂₂H₄₅ | 3 % AOETAC | 1 % HDDMA | - | 9,9 % Butylacrylat | a |
| 232 | 86,1 % C₂₂H₄,₅ | 3 % AOETAC | 1 % HDDMA | - | 9,9 % Hexadecylacrylat | a |
| 233 | 86,1 % C₂₂H₄₅ | 3 % AOETAC | 1 % HDDMA | - | 9,9 % Octyldodecylacrylat | a |
| 234 | 86,1 % C₂₂H₄₅ | 3 % AOETAC | 1 % HDDMA | - | 9,9 % Isostearylacrylat | a |
| 235 | 86,1 % C₂₂H₄₅ | 3 %AOETAC | 1 % HDDMA | - | 9,9 % C₂₆-C₂₈ α-Olefin | a |
| 236 | 86,1 % C₂₂H₄₅ | 3 % AOETAC | 1 % HDDMA | - | 9,9 % C₃₀ α-Olefin | a |
| 237 | 97 % C₂₂H₄₅ | 3 % MAPTAC | - | - | - | a |
| 238 | 97 % C₂₂H₄₅ | 2 % MAPTAC | 1 % HDDMA | - | - | a |
| 239 | 97 % C₂₂H₄₅ | 2 % MAPTAC | 1 % DDDMA | - | - | a |
| 240 | 97 %C₂₂H₄₅ | 2 % MAPTAC | 1 % TMPTA | - | - | a |
| 241 | 97 % C₂₂H₄₅ | 2 % MAPTAC | 1 % HDDMA/TMPTA | - | - | a |
| 242 | 87 % C₂₂H₄₅ | 3 % MAPTAC | - | 10 % B250MA | - | a |
| 243 | 86 % C₂₂H₄₅ | 3 % MAPTAC | 1 % HDDMA | 10 % B250MA | - | a |
| 244 | 78,7 % C₂₂H₄₅ | 2,7% MAPTAC | 4,8% HDDMA | 9 % B250MA | 4,8 % Butylacrylat | a |
| 245 | 78,7 % C₂₂H₄₅ | 2,7% MAPTAC | 4,8% HDDMA | 9 % B250MA | 4,8 % Hexadecylacrylat | a |
| 246 | 78,7 % C₂₂H₄₅ | 2,7% MAPTAC | 4,8% HDDMA | 9 % B250MA | 4,8 % Octyldodecylacrylat | a |
| 247 | 78,7 % C₂₂H₄₅ | 2,7% MAPTAC | 4,8% HDDMA | 9 % B250MA | 4,8 % Isostearylacrylat | a |
| 248 | 78,7 % C₂₂H₄₅ | 2,7% MAPTAC | 4,8% HDDMA | 9 % B250MA | 4,8 % C₂₈-C₂₈ α-Olefin | a |
| 249 | 78,7 % C₂₂H₄₅ | 2,7% MAPTAC | 4,8% HDDMA | 9 % B250MA | 4,8 % C₃₀ α-Olefin | a |
| 250 | 78,7 % C₂₂H₄₅ | 2,7% MAPTAC | 4,8% DDDMA | 9 % B250MA | 4,8 % C₂₆-C₂₈ α-Olefin | a |
| 251 | 78,7 % C₂₂H₄₅ | 2,7% MAPTAC | 4,8% DDDMA | 9 % B250MA | 4,8 % C₃₀ α-Olefin | a |
| 252 | 78,7 % C₂₂H₄₅ | 2,7% MAPTAC | 4,8% TMPTA | 9 % B250MA | 4,8 % C₂₆-C₂₈ α-Otefin | a |
| 253 | 78,7 % C₂₂H₄₅ | 2,7% MAPTAC | 4,8% TMPTA | 9 % B250MA | 4,8 % C₃₀ α-Olefin | a |
| 254 | 78,7 % C₂₂H₄₅ | 3 % MAPTAC | 1 % HDDMA/TMPTA | 10 % B250MA | - | a |
| 255 | 78,7 % C₂₂H₄₅ | 2,7% MAPTAC 1 | 4,8% HDDMA/TMPTA | 9 % B250MA | 4,8 % C₂₆-C₂₈ α-Olefin | a |
| 256 | 78,7 % C₂₂H₄₅ | 3 % MAPTAC | 1 % HDDMA | 10 % T250MA | - | a |
| 257 | 78,7 % C₂₂H₄₅ | 3 %MAPTAC | 1 % HDDMA | 10 % T080MA | - | a |
| 258 | 78,7 % C_{ZZ}H₄₅ | 3 % MAPTAC | 1 % HDDMA | 10 % LA070MA | - LA070MA - | a |
| 259 | 86,1 % C₂₂H₄₅ | 3 % MAPTAC | 1 % HDDMA | - | 9,9 % Butylacrylat | a |
| 260 | 86,1 % C₂₂H₄₅ | 3 % MAPTAC | 1 % HDDMA | - | 9,9 % Hexadecylacrylat | a |
| 261 | 86,1 % C₂₂H₄₅ | 3 % MAPTAC | 1 % HDDMA | - | 9,9 % Octyldodecylacrylat | a |
| 262 . | 86,1 % C₂₂H₄₅ | 3 % MAPTAC | 1 % HDDMA | - | 9,9 % Isostearylacrylat | a |
| 263 | 86,1%C₂₂H₄₅ | 3 % MAPTAC | 1 % HDDMA | - | 9,9 % C₂₆-C₂₈ α-Olefin | a |
| 264 | 86,1 % C₂₂H₄₅ | 3 % MAPTAC | 1 % HDDMA | - | 9,9 % C₃₀ α-Olefin | a |
| 265 | 78,2 % C₂₂H₄₅ | 3,6% AOETAC/ MAPTAC | 4,7% HDDMA/TMPTA | 9 % B250MA | 4,7 % C₂₆-C₂₈ α-Olefin | a |
| 266 | 97 % C₂₂H₄₅ | 3 % DADMAC | DADMAC - | - | - | a |
| 267 | 97 % C₂₂H₄₅ | 2 % DADMAC | 1 % HDDMA | - | - | a |
| 268 | 97 % C₂₂H₄₅ | 2 % DADMAC | 1 % DDDMA | - | - | a |
| 269 | 97 % C₂₂H₄₅ | 2 % DADMAC | 1 % TMPTA | - | - | a |
| 270 | 86 % C₂₂H₄₅ | 3 % DADMAC | 1 % HDDMA | 10 % B250MA | - | a |
| 271 | 78,7 % C₂₂H₄₅ | 2,7% DADMAC | 4,8% HDDMA | 9 % B250MA | 4,8 % C₂₆-C₂₈ α-Olefin | a |
| 272 | 78,7 % C₂₂H₄₅ | 2,7% DADMAC | 4,8% HDDMA | 9 % 8250MA | 4,8 % C₃₀ α-Olefin | a |
| 273 | 97 % C₁₈H₃₇ | 3 % AOETAC | AOETAC - | - | - | b1 |
| 274 | 92,4 % C₁₈H₃₇ | 2,8% AOETAC | 4,8% HDDMA | - | - | b1 |
| 275 | 92 % C₁₈H₃₇ | 3 % AOETAC | AOETAC - | - | 5 % Laurylmethacrylat | b1 |
| 276 | 87 % C₁₈H₃₇ | 3 % AOETAC | - | - | 10 % Laurylmethacrylat | b1 |
| 277 | 82 % C₁₈H₃₇ | 3 % AOETAC | | - | 15 % Laurylmethacrylat | b1 |
| 278 | 97 % C₁₈H₃₇ | 3 % AOETAC | | - | - | b2 |
| 279 | 92,4 % C₁₈H₃₇ | 2,8% AOETAC | 4,8% HDDMA | - | - | b2 |
| 280 | 86 % C₁₈H₃₇ | 3 % AOETAC | 1 % HDDMA | 10 % B250MA | - | b2 |
| 281 | 92 % C₁₈H₃₇ | 3 % AOETAC | - | 5 % B250MA | - | b2 |
| 282 | 87 % C₁₈H₃₇ | 3 % AOETAC | | 10 % B250MA | - | b2 |
| 283 | 82 % C₁₈H₃₇ | 3 % AOETAC | - | 15 % B250MA | - | b2 |
| 284 | 77 % C,₈H₃₇ | 3 % AOETAC | AOETAC - | 20 % B250MA | - | b2 |
| 285 | 78,7 % C₁₈H₃₇ | 2,7% AOETAC | 4,8% HDDMA | 9 % B250MA | 4,8 % C₂₆-C₂₆ α-Olefin | b2 |

Folgende kosmetische Formulierungen wurden mit erfindungsgemäßen Copolymeren hergestellt:

### Beispiel 1: W/O-Creme

| | | |
|---|---|---|
| A | Paraffinöl | 12.50 % |
| | Isopropylpalmitat | 7.50 % |
| | Sojabohnenöl | 5.00 % |
| | Magnesiumstearat | 1.00 % |
| | Copolymer Nr. 1 | 2.00 % |
| B | Wasser | ad 100 % |
| C | Konservierungsmittel | q.s. |

### Herstellung

I Schmelzen der Komponenten A bei 80°C
II Erwärmen von B auf 80 °C
III II in I einrühren
IV C bei 35 °C hinzugeben

### Beispiel 2: W/O-Creme

| | | |
|---|---|---|
| A | Decyloleat | 7.50 % |
| | Squalan | 7.50 % |
| | Velsan^{®} CCT | 7.50 % |
| | *Capryl-Caprinsäure- triglycerid* | |
| | Isopropylpalmitat | 7.50 % |
| | Magnesiumstearat | 0.67 % |
| | Aluminiumstearat | 0.33 % |
| | Copolymer Nr. 1 | 2.50 % |
| B | Wasser | ad 100 % |
| | 1,2-Propylenglykol | 2.00 % |
| | Magnesiumsulfat | 1.00 % |
| C | Konservierungsmittel | q.s. |
| | Tocopherolacetat | 0.10 % |

### Herstellung

I Schmelzen der Komponenten A bei 80 °C
II Mischen der Komponenten B und Erwärmen auf 80 °C
III II in I einrühren
IV C bei 35 °C hinzugeben

### Beispiel 3: W/O-Creme

| | | |
|---|---|---|
| A | Polyglyceryl-2-sesquiisostearat | 2.00 % |
| | Paraffinöl | 12.50 % |
| | Isopropylpalmitat | 7.50 % |
| | Sojabohnenöl | 5.00 % |
| | Magnesiumstearat | 1.00 % |
| | Copolymer Nr. 1 | 2.00 % |
| B | Wasser | ad 100 % |
| C | Konservierungsmittel | q.s. |

### Herstellung

I Schmelzen der Komponenten A bei 80 °C
II Erwärmen von Komponente B auf 80 °C
III II in I einrühren
IV C bei 35 °C hinzugeben

### Beispiel 4: W/O-Creme

| | | |
|---|---|---|
| A | Polyglyceryl-2-sesquüsostearat | 2.00 % |
| | Bienenwachs | 1.00 % |
| | Mikrokristallines Wachs | 1.00 % |
| | Paraffinöl | 12.50 % |
| | Isopropylpalmitat | 7.50 % |
| | Sojabohnenöl | 5.00 % |
| | Magnesiumstearat | 1.00 % |
| | Copolymer Nr. 1 | 1.00 % |
| B | Wasser | ad 100 |
| C | Konservierungsmittel | q.s. |

### Herstellung

I Schmelzen der Komponenten A bei 80 °C
II Erwärmen von Komponente B auf 80 °C
III II in I einrühren
IV C bei 35 °C hinzugeben

### Beispiel 5: W/O-Creme

| | | |
|---|---|---|
| A | Bienenwachs | 2.00 % |
| | Mikrokristallines Wachs | 3.00 % |
| | Paraffinöl | 12.50 % |
| | Isopropylpalmitat | 7.50 % |
| | Sojabohnenöl | 5.00 % |
| | Magnesiumstearat | 1.00 % |
| | Copolymer Nr. 1 | 1.00 % |
| B | Wasser | ad 100 |
| C | Konservierungsmittel | q.s. |

### Herstellung

I Schmelzen der Komponenten A bei 80 °C
II Erwärmen von Komponente B auf 80 °C
III II in I einrühren
IV C bei 35 °C hinzugeben

### Beispiel 6: Creme-Spülung

| | | |
|---|---|---|
| A | Hostacerin^{®} T3 | 1.50 % |
| | *Ceteareth- 3* | |
| | Cetylalkohol | 3.00 % |
| | Copolymer Nr. 30 | 1.00 % |
| | Genamin^{®} CTAC | 2.30 % |
| | *Cetrimonium Chloride* | |
| B | Wasser | ad 100 % |
| C | Zitronensäure | q.s. |

### Herstellung

I Schmelzen der Komponenten A bei 80 °C
II Zugabe von C zu B bis pH 3,7 und Erwärmung auf 80 °C
III II in I einrühren, bis Abkühlung auf 35 °C weiterrühren

### Beispiel 7: Creme-Spülung

| | | |
|---|---|---|
| A | Hostacerin^{®} T3 | 1.50 % |
| | *Ceteareth- 3* | |
| | Cetylalkohol | 3.00 % |
| | Copolymer Nr. 30 | 0.50 % |
| | Genamin^{®} CTAC | 1.65 % |
| | *Cetrimonium Chloride* | |
| B | Wasser | ad 100 % |
| C | Zitronensäure | q.s. |

### Herstellung

I Schmelzen der Komponenten A bei 80 °C
II Zugabe von C zu B bis pH 3,7
III Erwärmen von II auf 80 °C
IV III in I einrühren, bis Abkühlung auf 35 °C weiterrühren

### Beispiel 8: Sonnenschutz-Cremegel

| | | |
|---|---|---|
| A | Tegosoft^{®} TN | 8.00 % |
| | *C₁₂₋₁₅ Alkyl Benzoat* | |
| | Velsan^{®} CCT | 5.00 % |
| | *Capryl-Caprinsäure- triglycerid* | |
| | Eusolex^{®} OCR | 9.00 % |
| | *Octocrylen* | |
| | Eusolex^{®} 2292 | 7.00 % |
| | *Ethylhexyl Methoxycinnamat* | |
| | Eusolex^{®} 9020 | 2.50 % |
| | *Butyl Methoxydibenzoylmethan* | |
| | Cetearylalkohol | 1.00 % |
| | Emulsogen^{®} HCO 040 | 1.00 % |
| | *PEG-40 hydrogeniertes Rizinusöl* | |
| | Copolymer Nr. 1 | 2.00 % |
| | Phenonip^{®} | q.s. |
| | *Phenoxyethanol, Methylparaben, Ethylparaben, Butylparaben, Propylparaben, Isobutylparaben* | |
| B | Wasser | ad 100.00 % |

### Herstellung

I Schmelzen der Komponenten A bei 70 °C
II Erwärmen von Komponente B auf 70 °C
III II in I einrühren

### Beispiel 9: Creme-Spülung

| | | |
|---|---|---|
| A | Dow Corning^{®} 5200 | 1.00 % |
| | *Laurylmethicon Copolyol* | |
| | Cetylester | 1.00 % |
| | Cetearylalkohol | 3.00 % |
| B | Genamin^{®} KDMP | 0.50 % |
| | *Behentrimoniumchlorid* | |
| | Copolymer Nr. 30 | 1.00 % |
| C | Wasser | ad 100.00 % |
| | Konservierungsmittel | q.s. |
| | Hydrotriticum WQ | 1.00 % |
| D | Parfum | 0.30 % |
| | Farbstoff | q.s. |
| | Dow Corning^{®} 949 | 2.00 % |
| | *Amodimethicone* | |
| | *Cetrimoniumchlorid Trideceth-12* | |

### Herstellung

I Schmelzen der Komponenten A bei 75 °C
II B in C einrühren und auf 75 °C erwärmen
III II in I einrühren, bis Abkühlung auf 30 °C weiterrühren
IV D bei einer Temperatur von 30 °C zu III hinzugeben
V IV auf pH 4.0 einstellen

### Beispiel 10: Creme-Spülung

| | | |
|---|---|---|
| A | Hostacerin^{®} DGI | 1.50% |
| | *Polyglyceryl-2-Sesquiisostearate* | |
| | Cetylalkohol | 4.00% |
| B | Genamin^{®} BTLF | 2.30 % |
| | *Behentrimoniumchlorid* | |
| | Copolymer Nr. 30 | 1.00 % |
| | Wasser | ad 100 % |
| C | Parfum | 0.30 % |
| | Konservierungsmittel | q.s. |
| | Farbstoff | q.s. |
| | SilCare^{®} Silicone SEA | 1.00 % |
| | *Trideceth-9 PG-Amodimethicone Trideceth-12* | |

### Herstellung

I Schmelzen der Komponenten A bei 70 °C
II Erwärmung der Komponenten B auf 70 °C
III Einrühren von II in I, bis Abkühlung auf 30 °C weiterrühren
IV C bei 30 °C zu III hinzugeben
V IV auf pH 4.0 einstellen

### Beispiel 11: Kinder-Sonnenschutz

| | | |
|---|---|---|
| A | Abil^{®} EM 90 | 2.00% |
| | *Cetyl PEG*/*PPG-10*/*1 Dimethicon* | |
| | Dow Corning^{®} 246 | 11.00% |
| | *Cyclopentasiloxan*/ *Cyclohexasiloxan* | |
| | Titandioxid UV Titan M 262 | 10.00% |
| | *Titandioxid*/ *Aluminiumoxid*/ *Stearinsäure* | |
| | Crodamol AB | 12.00% |
| | *C12-15 Alkylbenzoat* | |
| | Zinkoxid HP1 | 4.00% |
| | *Zinkoxid* | |
| | 1,3-Butanediol | 3.00% |
| | *Butylenglycol* | |
| | Abil^{®} WE09 | 6.00% |
| | *Hexyllaurat*/ *Polyglyceryl-4 Isostearat*/ *Cetyldimethicon* | |
| | Copolymer Nr. 1 | 0.50% |
| | Aluminium/Magnesium | 0.50% |
| | Hydroxidstearat | |
| | Aluminiumstearat | 0.50% |
| | Cutina^{®} HR | 0.50% |
| | *hydrogeniertes Rizinusöl* | |
| B | Wasser | ad 100 % |
| | Glycerin | 5.00% |
| | Ginkgo Biloba Extrakt (flüssig) | 0.70% |
| | Sodium Carboxymethyl Betaglutan | 0.20% |
| | *Polyglucose* | |
| | Aquamollin BC plv. Hoko | 0.20% |
| | *Dinatrium-EDTA* | |
| C | Phenonip^{®} ME | 0.80% |
| | *Methyl-*/*Propylparaben*/ *Phenoxyethanol* | |
| | Natriumchlorid | 1.00% |
| | Aluminiumhydroxid | 0.30% |

### Herstellung

I Schmelzen der Komponenten A bei 80 °C
II Erwärmen der Komponenten B auf 80 °C
III Einrühren von II in I
IV C zu III nach Abkühlen auf 35 °C hinzufügen

### Beispiel 12: Paraffinölgel

| | |
|---|---|
| Copolymer Nr. 277 | 5.00% |
| Paraffinöl | 95.00% |

### Herstellung

- I: Mischen und Erwärmen der Komponenten bis zum Erhalt einer homogenen Lösung

Die Beurteilung des Gels erfolgte nach dem Abkühlen auf Raumtemperatur. Das erhaltene Ölgel war farblos und transparent bis leicht opak.

### Beispiel 13: Verdickung von Velsan^{®} CCT (Caprinsäure Caprylic Triglyceride, Clariant)

| | |
|---|---|
| Copolymer Nr. 60 | 5.00% |
| Velsan^{®} CCT | 95.00% |
| *Capryl-Caprinsäure- triglycerid* | |

### Herstellung

- I: Mischen und Erwärmen der Komponenten bis zum Erhalt einer homogenen Lösung

Die Beurteilung des Gels erfolgte nach dem Abkühlen auf Raumtemperatur. Das erhaltene Ölgel war amber und transparent bis leicht opak.

### Beispiel 14: Verdickung von Rapsölmethylester

| | |
|---|---|
| Copolymer Nr. 40 | 5.00% |
| Rapsölmethylester | 95.00% |

### Herstellung

- I: Mischen und Erwärmen der Komponenten bis zum Erhalt einer homogenen Lösung

Die Beurteilung des Gels erfolgte nach dem Abkühlen auf Raumtemperatur. Das erhaltene Ölgel war tief gelb und transparent bis leicht opak.

### Beispiel 15: Verdickung von einer Sonnenblumenöl / Sojaöl (23 : 77 Gew.-%) Mischung

| | |
|---|---|
| Copolymer Nr. 52 | 5.00% |
| Sonnenblumenöl / Sojaöl | 95.00% |

### Herstellung

- I: Mischen und Erwärmen der Komponenten bis zum Erhalt einer homogenen Lösung

Die Beurteilung des Gels erfolgte nach dem Abkühlen auf Raumtemperatur. Das erhaltene Ölgel war gelb und transparent bis leicht opak.

### Beispiel A

Die Formulierungsbeispiele 1 - 5, 8 und 11 wurden nachgestellt, wobei anstelle des Polymers Nr. 1 jeweils die Polymere Nr. 4, 25, 30, 31, 33, 39, 40, 52, 60, 111, 273, 275, 276, 277, 278 und 282 aus Tabelle A in gleicher Menge eingesetzt wurden. Diese Formulierungen wurden bei 40 °C eingelagert und über 6 Wochen bei dieser Temperatur gehalten. Es wurden stabile Formulierungen erhalten.

### Beispiel B

Die Formulierungsbeispiele 6, 7, 9 und 10 wurden nachgestellt, wobei anstelle des Polymers Nr. 30 die Polymere Nr. 1, 4, 25, 31, 33, 39, 40, 52, 60, 111, 273, 275, 276, 277, 278 und 282 aus Tabelle A in gleicher Menge eingesetzt wurden. Es wurden homogene Creme Spülungen erhalten.

### Beispiel C

In den Formulierungsbeispielen 12 - 15 wurden alternativ 2 Gew.% an den entsprechenden Polymeren und 98 Gew.% an Öl eingesetzt. Auch mit dem verringerten Anteil an Polymer konnten gute bis sehr gute Verdickungseigenschaften erzielt werden.

### Beispiel D

Die Formulierungsbeispiele 12-15 wurden nachgestellt, wobei in Formulierungsbeispiel 12 anstelle des Polymers Nr. 277 die Polymere Nr. 1, 4, 25, 30, 31, 33, 39, 40, 52, 60, 111, 273, 275, 276, 278 und 282, in Formulierungsbeispiel 13 anstelle des Polymers Nr. 60 die Polymere Nr. 1, 4, 25, 30, 31, 33, 39, 40, 52, 111, 273, 275, 276, 277, 278 und 282, in Formulierungsbeispiel 14 anstelle des Polymers Nr. 40 die Polymere Nr. 1, 4, 25, 30, 31, 33, 39, 52, 60, 111, 273, 275, 276, 277, 278 und 282, in Formulierungsbeispiel 15 anstelle des Polymers Nr. 52 die Polymere Nr. 1, 4, 25, 30, 31, 33, 39, 40, 60, 111, 273, 275, 276, 277, 278 und 282 aus Tabelle A in gleicher Menge eingesetzt wurden. Die Ölgele wurden visuell bei 20 °C als klar und transparent bis leicht opak beurteilt.

### Beispiel E

Es wurden die Formulierungsbeispiele aus Beispiel D nachgestellt wobei jedoch anstelle von 5 Gew.-% des entsprechenden Polymers jeweils 2 Gew.-% Polymer und 98 Gew.-% Öl eingesetzt wurden. Auch mit dem verringerten Anteil an Polymer konnten gute bis sehr gute Verdickungseigenschaften erzielt werden.

### Beispiel F

Die Formulierungsbeispiele aus Beispiel A - E wurden nachgestellt, wobei anstelle der in den Beispielen A - E eingesetzten Polymere die Polmere Nr. 121, 123, 132, 135, 136, 140, 141, 145, 147, 189, 197, 199, 208, 211, 212, 216, 217, 221, 223 und 265 aus Tabelle A in gleicher Menge eingesetzt wurden.

Chemische oder INCI- Bezeichnung der verwendeten Abkürzungen oder Handelsnamen
- AMA: Allylmethacrylat
- AOETAC: 2-(Acryloyloxy)ethyl]trimethylammoniumchlorid
- APTAC: [3-Acrylamidopropyl]trimethylammoniumchlorid
- B250MA: Behenylmethacrylat, ethoxyliert mit 25 EO-Einheiten
- BDDMA: Butandioldimethacrylat
- DADMAC: Diallyldimethylammoniumchlorid
- DDDMA: Dodecandioldimethacrylat
- EDDMA: Ethandioldimethacrylat
- HDDMA: Hexandioldimethacrylat
- HEMA: 2-Hydroxyethylmethacrylat
- LA070MA: Laurylmethacrylat, ethoxyliert mit 7 EO-Einheiten
- MAOETAC: [2-(Methacryloyloxy)ethyl]trimethylammoniumchlorid
- MAPTAC: [3-Methacrylamidopropyl]trimethylammoniumchlorid
- MBA: Methylen-bis-acrylamid
- PEG-Diacrylat: Polyethylenglykol-Diacrylat mit einem durchschnittlichen Molekulargewicht Mₙ = 575
- Poly-NVP: Poly-N-Vinylpyrrolidon
- T080MA: Talylmethacrylat, ethoxyliert mit 8 EO-Einheiten
- T250MA: Talylmethacrylat, ethoxyliert mit 25 EO-Einheiten
- TEMPO: 2,2,6,6-Tetramethylpiperidinyloxyl
- TMPTA: Trimethylolpropantriacrylat
- TMPTMA: Trimethylolpropantrimethacrylat

## Patentansprüche

1. Copolymer enthaltend
a) 60,0 - 99,9 Gew.-% an einer oder mehreren Struktureinheiten hervorgegangen aus polymerisierbaren Substanzen der folgenden Strukturformel (I) 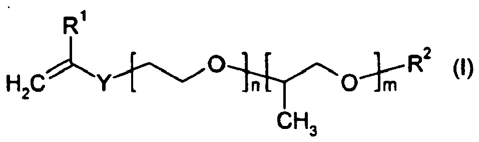 worin
R¹ Wasserstoff oder Methyl bedeutet,
Y O, NR³, S, CH₂O, CH₂NR³, CH₂S, C(O), C(NR³), C(O)O oder C(O)NR³ bedeutet,
R² einen linearen oder verzweigten gesättigten Alkylrest mit 12 bis 200 C-Atomen oder einen linearen oder verzweigten ein- oder mehrfach ungesättigten Alkenylrest mit 12 bis 200 C-Atomen bedeutet,
R³ Wasserstoff oder Methyl bedeutet und
n und m jeweils unabhängig voneinander eine ganze Zahl von 0 bis 200 bedeuten,
b) 0,1 - 20,0 Gew.% an einer oder mehreren Struktureinheiten hervorgegangen aus polymerisierbaren quaternären Ammoniumverbindungen, und
c) 0 - 20,0 Gew.-% an einer oder mehreren nichtionischen Struktureinheiten hervorgegangen aus einer oder mehreren weiteren polymerisierbaren Substanzen.

2. Copolymer nach Anspruch 1, **dadurch gekennzeichnet, dass** es 60,0 bis 99,9 Gew.% an einer oder mehreren Struktureinheiten der Komponente a) enthält, die hervorgegangen sind aus polymerisierbaren Substanzen der folgenden Strukturformel (I) 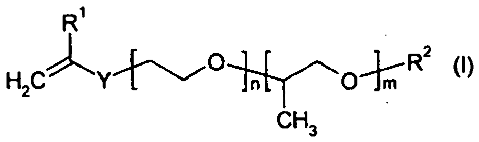 worin
R¹ Wasserstoff oder Methyl bedeutet,
Y O, NR³, CH₂O, CH₂NR³, C(O)O oder C(O)NR³ und bevorzugt C(O)O oder C(O)NR³ bedeutet,
R² einen linearen oder verzweigten gesättigten Alkylrest mit 12 bis 200, bevorzugt mit 12 bis 58, besonders bevorzugt mit 12 bis 40 und insbesondere bevorzugt mit 12 bis 30 C-Atomen oder einen linearen oder verzweigten ein- oder mehrfach ungesättigten Alkenylrest mit 12 bis 200, bevorzugt mit 12 bis 58, besonders bevorzugt mit 12 bis 40 und insbesondere bevorzugt mit 12 bis 30 C-Atomen bedeutet,
R³ Wasserstoff oder Methyl bedeutet und
n und m jeweils unabhängig voneinander eine ganze Zahl von 0 bis 200, bevorzugt von 0 bis 100, besonders bevorzugt von 0 bis 50 und insbesondere bevorzugt von 0 bis 30 bedeuten.

3. Copolymer nach Anspruch 1 oder 2, **dadurch gekennzeichnet, dass** es 60,0 bis 99,9 Gew.-% an einer oder mehreren Struktureinheiten der Komponente a) enthält, die hervorgegangen sind aus einer oder mehreren polymerisierbaren Verbindungen ausgewählt aus den Strukturformeln (Ia) und (Ib) 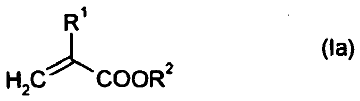 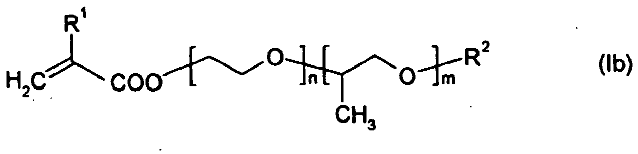 worin
R¹ Wasserstoff oder Methyl bedeutet,
R² einen linearen oder verzweigten gesättigten Alkylrest mit 12 bis 200, bevorzugt mit 12 bis 58, besonders bevorzugt mit 12 bis 40 und insbesondere bevorzugt mit 12 bis 30 C-Atomen oder einen linearen oder verzweigten ein- oder mehrfach ungesättigten Alkenylrest mit 12 bis 200, bevorzugt mit 12 bis 58, besonders bevorzugt mit 12 bis 40 und insbesondere bevorzugt mit 12 bis 30 C-Atomen, bedeutet und
n und m jeweils unabhängig voneinander eine ganze Zahl von 0 bis 200, bevorzugt von 0 bis 100, besonders bevorzugt von 0 bis 50 und insbesondere bevorzugt von 0 bis 30 bedeuten und die Summe n + m eine Zahl von 1 bis 200, bevorzugt von 2 bis 100, besonders bevorzugt von 3 bis 50 und insbesondere bevorzugt von 3 bis 30, ist.

4. Copolymer nach Anspruch 3, **dadurch gekennzeichnet, dass** die Verbindungen der Formel (Ia) ausgewählt sind aus Laurylacrylat, Laurylmethacrylat, Hexadecylacrylat, Hexadecylmethacrylat, Stearylacrylat, Stearylmethacrylat, Isostearylacrylat, Isotearytmethacrylat, Octyldodecylacrylat, Octyldodecylmethacrylat, Behenylacrylat und Behenylmethacrylat und die Verbindungen der Formel (Ib) ausgewählt sind aus Acrylsäureester von Laurylalkohol mit 7 EO-Einheiten, Methacrylsäureester von Laurylalkohol mit 7 EO-Einheiten, Acrylsäureester von Talylalkohol mit 8 EO-Einheiten, Methacrylsäureester von Talylalkohol mit 8 EO-Einheiten, Acrylsäureester von Talylalkohol mit 25 EO-Einheiten, Methacrylsäureester von Talylalkohol mit 25 EO-Einheiten, Acrylsäureester von Behenylalkohol mit 25 EO-Einheiten und Methacrylsäureester von Behenylalkohol mit 25 EO-Einheiten.

5. Copolymer nach einem oder mehreren der Ansprüche 1 bis 4, **dadurch gekennzeichnet, dass** R² in den Verbindungen der Formel (I) oder in den Verbindungen der Formeln (Ia) und (Ib) einen linearen oder verzweigten, vorzugsweise einen linearen, gesättigten Alkylrest mit 12 bis 200, bevorzugt mit 12 bis 58, besonders bevorzugt mit 12 bis 40 und insbesondere bevorzugt mit 12 bis 30 C-Atomen bedeutet.

6. Copolymer nach einem oder mehreren der Ansprüche 1 bis 5, **dadurch gekennzeichnet, dass** die Struktureinheiten der Komponente b) hervorgegangen sind aus Substanzen ausgewählt aus
b1) polymerisierbaren quaternären Ammoniumverbindungen der folgenden Strukturformel (II)
X⁻⁺NR⁷R⁸R⁹R¹⁰ (II)
worin
X⁻ ein negativ geladenes Gegenion bedeutet,
R⁷ Vinyl-, Allyl- oder einen Rest der folgenden Strukturformel (III) 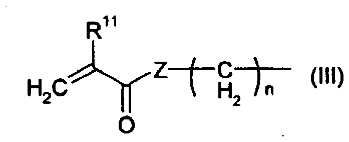
bedeutet, worin
R¹¹ Wasserstoff oder Methyl bedeutet,
Z O, NH, NCH₃ oder S bedeutet,
n eine ganze Zahl von 1 bis 50 bedeutet,
R⁸ einen linearen oder verzweigten Alkylrest mit 1 bis 50 C-Atomen oder einen Rest R⁷ bedeutet,
R⁹ einen linearen oder verzweigten Alkylrest mit 1 bis 50 C-Atomen oder einen Rest R⁷ bedeutet und
R¹⁰ einen linearen oder verzweigten Alkylrest mit 1 bis 50 C-Atomen bedeutet
und
b2) polymerisierbaren cyclischen quaternären Ammoniumverbindungen der folgenden Strukturformel (IV) 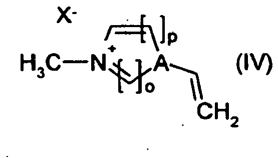 worin
o und p jeweils unabhängig voneinander eine ganze Zahl von 0 bis 5 bedeuten,
A C, CH, N oder P bedeutet und
X⁻ ein negativ geladenes Gegenion bedeutet.

7. Copolymer nach Anspruch 6, **dadurch gekennzeichnet, dass** die negativ geladenen Gegenionen X⁻ in Formel (II) und (IV) ausgewählt sind aus Chlorid, Bromid, Iodid, ½ SO₄²⁻ und Methosulfat.

8. Copolymer nach einem oder mehreren der Ansprüche 1 bis 7, **dadurch gekennzeichnet, dass** die Struktureinheiten der Komponente b) hervorgegangen sind aus polymerisierbaren quaternären Ammoniumverbindungen ausgewählt aus Diallyldimethylammoniumchlorid (DADMAC), [2-(Methacryloyloxy)ethyl]-trimethylammoniumchlorid (MAOETAC), [2-(Acryloyloxy)ethyl]-trimethylammoniumchlorid (AOETAC), [2-Methacrylamidoethyl]trimethylammoniumchlorid, [2-(Acrylamido)ethyl]trimethylammoniumchlorid, [3-Methacryl-amidopropyl]trimethylammoniumchlorid (MAPTAC), [3-Acrylamidopropyl]trimethylammoniumchlorid (APTAC), N-Methyl-2-vinylpyridiniumchlorid, N-Methyl-4-vinyl-pyridiniumchlorid und N-Methyl-3-vinylimidazoliniumchlorid.

9. Copolymer nach einem oder mehreren der Ansprüche 6 bis 8, **dadurch gekennzeichnet, dass** die Struktureinheiten der Komponente b) hervorgegangen sind aus polymerisierbaren quaternären Ammoniumverbindungen der Strukturformel (II).

10. Copolymer nach einem oder mehreren der Ansprüche 1 bis 9, **dadurch gekennzeichnet, dass** es ein oder mehrere Struktureinheiten der Komponente c) enthält, die hervorgegangen sind aus Substanzen ausgewählt aus Monomeren mit mehr als einer polymerisierbaren Gruppe, die vernetzend wirken können.

11. Copolymer nach einem oder mehreren der Ansprüche 1 bis 10, **dadurch gekennzeichnet, dass** die Struktureinheiten der Komponente c) hervorgegangen sind aus Substanzen ausgewählt aus Divinylbenzol, Methylenbisacrylamid (MBA), Triallylamin, Triallylcyanurat, Ethandioldiacrylat, Ethandioldimethacrylat (EDDMA), Butandioldiacrylat, Butandioldimethacrylat (BDDMA), Hexandioldiacrylat, Hexandioldimethacrylat (HDDMA), Dodecandioldiacrylat, Dodecandioldimethacrylat (DDDMA), Trimethylolpropantriacrylat (TMPTA), Trimethylolpropantrimethacrylat (TMPTMA), Glycerindiacrylat, Glycerindimethacrylat, Tetraethylenglykoldiacrylat, Tetraethylenglykoldimethacrylat, Poyethylenglykoldiacrylat mit Molmassen zwischen 400 und 800 g/mol, Poyethylenglykoldimethacrylat mit Molmassen zwischen 400 und 800 g/mol, Acryl-, Methacryl- oder Ethacrylsäureallylester, bevorzugt Acryl- oder Methacrylsäureallylester und besonders bevorzugt Methacrylsäureallylester (AMA), Dipropylenglykoldiallylether, Polyglykoldiallylether, Hydrochinondiallylether, Trimethylolpropandiallylether, Trimethylolpropantriallylether, Tetraallyloxyethan, Triethylenglykoldivinylether und anderen Allyl- oder Vinylethern multifunktioneller Alkohole, sowie aus Mischungen der vorgenannten Substanzen.

12. Copolymer nach einem oder mehreren der Ansprüche 1 bis 11, **dadurch gekennzeichnet, dass** die Menge an Struktureinheiten der Komponente a) von 60,0 bis 99,5 Gew.-%, die Menge an Struktureinheiten der Komponente b) von 0,5 bis 20,0 Gew.-% und die Menge an Struktureinheiten der Komponente c) von 0 bis 20,0 Gew.-% beträgt.

13. Copolymer nach einem oder mehreren der Ansprüche 1 bis 12, **dadurch gekennzeichnet, dass** die Menge an Struktureinheiten der Komponente a) von 80,0 bis 98,0 Gew.-%, die Menge an Struktureinheiten der Komponente b) von 0,7 bis 11,0 Gew.-% und die Menge an Struktureinheiten der Komponente c) von 0 bis 11,0 Gew.% beträgt.

14. Copolymer nach einem oder mehreren der Ansprüche 3 bis 5, **dadurch gekennzeichnet, dass** das Gewichtsverhältnis an Struktureinheiten hervorgegangen aus den Verbindungen der Formel (Ia) : Struktureinheiten hervorgegangen aus den Verbindungen der Formel (Ib) in den Copolymeren 1,0 : 0 bis 0,8 ist.

15. Copolymer nach einem oder mehreren der Ansprüche 1 bis 14, **dadurch gekennzeichnet, dass** es keine Struktureinheiten enthält, die aus anionischen polymerisierbaren Substanzen hervorgegangen sind.

16. Copolymer nach einem oder mehreren der Ansprüche 1 bis 15, **dadurch gekennzeichnet, dass** es aus den unter den Komponenten a) und b) genannten Struktureinheiten besteht.

17. Copolymer nach einem oder mehreren der Ansprüche 1 bis 15, **dadurch gekennzeichnet, dass** es aus den unter den Komponenten a), b) und c) genannten Struktureinheiten besteht.

18. Copolymer nach Anspruch 17, **dadurch gekennzeichnet, dass** die Struktureinheiten der Komponente c) aus vernetzenden Struktureinheiten ausgewählt sind.

19. Verfahren zur Herstellung eines Copolymers nach einem oder mehreren der Ansprüche 1 bis 18, **dadurch gekennzeichnet, dass** die Monomere radikalisch polymerisiert werden.

20. Kosmetische, pharmazeutische oder dermatologische Formulierung enthaltend ein oder mehrere Copolymere gemäß einem oder mehreren der Ansprüche 1 bis 18.

21. Verwendung eines oder mehrerer der Copolymere nach einem oder mehreren der Ansprüche 1 bis 18 zur Verdickung von Öl.

22. Verwendung eines oder mehrerer der Copolymere nach einem oder mehreren der Ansprüche 1 bis 18 zur Konditionierung von Fasern, vorzugsweise keratinischen Fasern.

23. Verwendung eines oder mehrerer der Copolymere nach einem oder mehreren der Ansprüche 1 bis 18 zur Erhöhung der Wasserfestigkeit von Sonnenschutzformulierungen.

24. Verwendung eines oder mehrerer der Copolymere nach einem oder mehreren der Ansprüche 1 bis 18 zur Erhöhung des Sonnenschutzfaktors von Sonnenschutzformulierungen.

25. Verwendung eines oder mehrerer der Copolymere nach einem oder mehreren der Ansprüche 1 bis 18 als Emulgator.

## Claims

1. Copolymer comprising
a) 60.0% - 99.9% by weight of one or more structural units originating from polymerizable substances of the following structural formula (I) 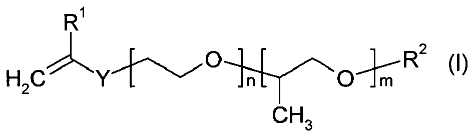 in which
R¹ is hydrogen or methyl,
Y is O, NR³, S, CH₂O, CH₂NR³, CH₂S, C(O), C(NR³), C(O)O or C(O)NR³,
R² is a linear or branched saturated alkyl radical, having 12 to 200 carbon atoms, or a linear or branched mono- or polyunsaturated alkenyl radical having 12 to 200 carbon atoms,
R³ is hydrogen or methyl, and
n and m each independently of one another are an integer from 0 to 200,
b) 0.1% - 20.0% by weight of one or more structural units, originating from polymerizable quaternary ammonium compounds, and
c) 0% - 20.0% by weight of one or more nonionic structural units originating from one or more further polymerizable substances.

2. Copolymer according to Claim 1, **characterized in that** it comprises 60.0% to 99.9% by weight of one or more structural units of component a) originating from polymerizable substances of the following structural formula (I) 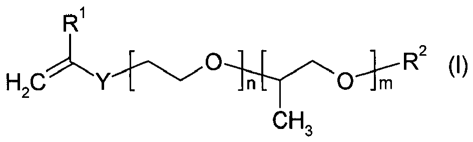 in which
R¹ is hydrogen or methyl,
Y is O, NR³, CH₂O, CH₂NR³, C(O)O or C(O)NR³, and preferably C(O)O or C(O)NR³,
R² is a linear or branched saturated alkyl radical having 12 to 200, preferably having 12 to 58, more preferably having 12 to 40, and with particular preference having 12 to 30 carbon atoms, or a linear or branched mono- or polyunsaturated alkenyl radical having 12 to 200, preferably having 12 to 58, more preferably having 12 to 40, and with particular preference having 12 to 30 carbon atoms,
R³ is hydrogen or methyl, and
n and m each independently of one another are an integer from 0 to 200, preferably from 0 to 100, more preferably from 0 to 50, and with particular preference from 0 to 30.

3. Copolymer according to claim 1 or 2, **characterized in that** it comprises 60.0% to 99.9% by weight of one or more structural units of component a) originating from one or more polymerizable compounds selected from the structural formulae (Ia) and (Ib) 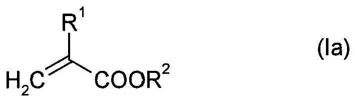 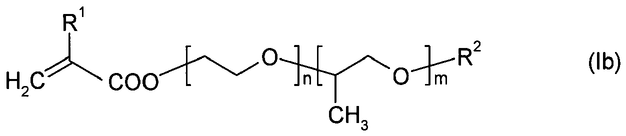 in which
R¹ is hydrogen or methyl, and
R² is a linear or branched saturated alkyl radical having 12 to 200, preferably having 12 to 58, more preferably having 12 to 40, and with particular preference having 12 to 30 carbon atoms, or a linear or branched mono- or polyunsaturated alkenyl radical having 12 to 200, preferably having 12 to 58, more preferably having 12 to 40, and with particular preference having 12 to 30 carbon atoms, and
n and m each independently of one another are an integer from 0 to 200, preferably from 0 to 100, more preferably from 0 to 50, and with particular preference from 0 to 30, and the sum n + m is a number from 1 to 200, preferably from 2 to 100, more preferably from 3 to 50, and with particular preference from 3 to 30.

4. Copolymer according to Claim 3, **characterized in that** the
compounds of the formula (Ia) are selected from lauryl acrylate, lauryl methacrylate, hexadecyl acrylate, hexadecyl methacrylate, stearyl acrylate, stearyl methacrylate, isostearyl acrylate, isostearyl methacrylate, octyldodecyl acrylate, octyldodecyl methacrylate, behenyl acrylate and behenyl methacrylate, and the compounds of the formula (Ib) are selected from acrylic ester of lauryl alcohol with 7 EO units, methacrylic ester of lauryl alcohol with 7 EO units, acrylic ester of talyl alcohol with 8 EO units, methacrylic ester of talyl alcohol with 8 EO units, acrylic ester of talyl alcohol with 25 EO units, methacrylic ester of talyl alcohol with 25 EO units, acrylic ester of behenyl alcohol with 25 EO units, and methacrylic ester of behenyl alcohol with 25 EO units.

5. Copolymer according to one or more of Claims 1 to 4, **characterized in that** R² in the compounds of the formula (I) or in the compounds of the formulae (Ia) and (Ib) is a linear or branched, preferably a linear, saturated alkyl radical having 12 to 200, preferably having 12 to 58, more preferably having 12 to 40, and with particular preference having 12 to 30 carbon atoms.

6. Copolymer according to one or more of Claims 1 to 5, **characterized in that** the structural units of component b) originate from substances selected from
b1) polymerizable quaternary ammonium compounds of the following structural formula (II)
X-⁺NR⁷R⁸R⁹R¹⁰ (II)
in which
X⁻ is a negatively charged counterion,
R⁷ is vinyl radical, allyl radical or a radical of the following structural formula (III) 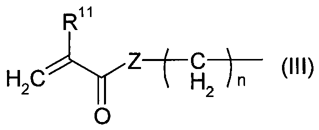
in which
R¹¹ is hydrogen or methyl,
Z is O, NH, NCH₃ or S,
n is an integer from 1 to 50,
R⁸ is a linear or branched alkyl radical having 1 to 50 carbon atoms or is a radical R⁷,
R⁹ is a linear or branched alkyl radical having 1 to 50 carbon atoms or is a radical R⁷, and
R¹⁰ is a linear or branched alkyl radical having 1 to 50 carbon atoms,
and
b2) polymerizable cyclic quaternary ammonium compounds with the following structural formula (IV) 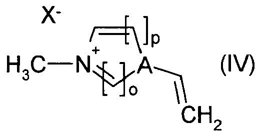 in which
o and p each independently of one another are an integer from 0 to 5,
A is C, CH, N or P, and
X⁻ is a negatively charged counterion.

7. Copolymer according to Claim 6, **characterized in that** the negatively charged counterions X⁻ in formula (II) and (IV) are selected from chloride, bromide, iodide, ½ SO₄²⁻ and methosulfate.

8. Copolymer according to one or more of Claims 1 to 7, **characterized in that** the structural units of component b) originate from polymerizable quaternary ammonium compounds selected from diallyldimethylammonium chloride (DADMAC), [2-(methacryloyloxy)ethyl]trimethylammonium chloride (MAOETAC), [2-(acryloyloxy)ethyl]trimethylammonium chloride (AOETAC), [2-methacrylamidoethyl]trimethylammonium chloride, [2-(acrylamido)ethyl]trimethylammonium chloride, [3-methacrylamidopropyl]trimethylammonium chloride (MAPTAC), [3-acrylamidopropyl]trimethylammonium chloride (APTAC), N-methyl-2-vinylpyridinium chloride, N-methyl-4-vinylpyridinium chloride, and N-methyl-3-vinylimidazolinium chloride.

9. Copolymer according to one or more of Claims 6 to 8, **characterized in that** the structural units of component b) originate from polymerizable quaternary ammonium compounds of the structural formula (II).

10. Copolymer according to one or more of Claims 1 to 9, **characterized in that** it comprises one or more structural units of component c) that originate from substances selected from monomers having more than one polymerizable group, which may have a crosslinking effect.

11. Copolymer according to one or more of Claims 1 to 10, **characterized in that** the structural units of component c) originate from substances selected from divinylbenzene, methylenebisacrylamide (MBA), triallylamine, triallyl cyanurate, ethanediol diacrylate, ethanediol dimethacrylate (EDDMA), buanediol diacrylate, buanediol dimethacrylate (BDDMA), hexanediol diacrylate, hexanediol dimethacrylate (HDDMA), dodecanediol diacrylate, dodecanediol dimethacrylate (DDDMA), trimethylolpropane triacrylate (TMPTA), trimethylolpropane trimethacrylate (TMPTMA), glycerol diacrylate, glycerol dimethacrylate, tetraethylene glycol diacrylate, tetraethylene glycol dimethacrylate, polyethylene glycol diacrylate with molar masses between 400 and 800 g/mol, polyethylene glycol dimethacrylate with molar masses between 400 and 800 g/mol, allyl acrylate, methacrylate or ethacrylate, preferably allyl acrylate or methacrylate, and more particularly allyl methacrylate (AMA), dipropylene glycol diallyl ether, polyglycol diallyl ether, hydroquinone diallyl ether, trimethylolpropane diallyl ether, trimethylolpropane triallyl ether, tetraallyloxyethane, triethylene glycol divinyl ether, and other allyl ethers or vinyl ethers of polyfunctional alcohols, and also from mixtures of the aforesaid substances.

12. Copolymer according to one or more of Claims 1 to 11, **characterized in that** the amount of structural units of component a) is from 60.0% to 99.5% by weight, the amount of structural units of component b) is from 0.5% to 20.0% by weight, and the amount of structural units of component c) is from 0% to 20.0% by weight.

13. Copolymer according to one or more of Claims 1 to 12, **characterized in that** the amount of structural units of component a) is from 80.0% to 98.0% by weight, the amount of structural units of component b) is from 0.7% to 11.0% by weight, and the amount of structural units of component c) is from 0% to 11.0% by weight.

14. Copolymer according to one or more of Claims 3 to 5, **characterized in that** the weight ratio of structural units originating from the compounds of the formula (Ia): structural units originating from the compounds of the formula (Ib) in the copolymers is 1.0 : 0 to 0.8.

15. Copolymer according to one or more of Claims 1 to 14, **characterized in that** it comprises no structural units originating from anionic polymerizable substances.

16. Copolymer according to one or more of Claims 1 to 15, **characterized in that** it consists of the structural units stated under components a) and b).

17. Copolymer according to one or more of Claims 1 to 15, **characterized in that** it consists of the structural units stated under components a), b), and c).

18. Copolymer according to Claim 17, **characterized in that** the structural units of component c) are selected from crosslinking structural units.

19. Process for preparing a copolymer according to one or more of Claims 1 to 18, **characterized in that** the monomers are radically polymerized.

20. Cosmetic, pharmaceutical or dermatological formulation comprising one or more copolymers according to one or more of Claims 1 to 18.

21. Use of one or more of the copolymers according to one or more of Claims 1 to 18 for thickening oil.

22. Use of one or more of the copolymers according to one or more of Claims 1 to 18 for conditioning fibers, preferably keratinic fibers.

23. Use of one or more of the copolymers according to one or more of Claims 1 to 18 for increasing the water resistance of sun protection formulations.

24. Use of one or more of the copolymers according to one or more of Claims 1 to 18 for increasing the sun protection factor of sun protection formulations.

25. Use of one or more of the copolymers according to one or more of Claims 1 to 18 as emulsifier.

## Revendications

1. Copolymère contenant :
a) 60,0 à 99,9 % en poids d'une ou de plusieurs unités structurales provenant de substances polymérisables de la formule structurale (I) suivante : 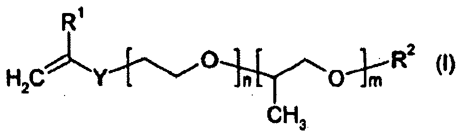 dans laquelle
R¹ signifie hydrogène ou méthyle,
Y signifie O, NR³, S, CH₂O, CH₂NR³, CH₂S, C(O), C(NR³), C(O)O ou C(O)NR³,
R² signifie un radical alkyle saturé linéaire ou ramifié contenant 12 à 200 atomes C ou un radical alcényle mono- ou polyinsaturé linéaire ou ramifié contenant 12 à 200 atomes C,
R³ signifie hydrogène ou méthyle, et
n et m signifient chacun indépendamment l'un de l'autre un nombre entier de 0 à 200,
b) 0,1 à 20,0 % en poids d'une ou de plusieurs unités structurales provenant de composés d'ammonium quaternaires polymérisables, et
c) 0 à 20,0 % en poids d'une ou de plusieurs unités structurales non ioniques provenant d'une ou de plusieurs substances polymérisables supplémentaires.

2. Copolymère selon la revendication 1, **caractérisé en ce qu'**il contient 60,0 à 99,9 % en poids d'une ou de plusieurs unités structurales du composant a), qui proviennent de substances polymérisables de la formule structurale (I) suivante : 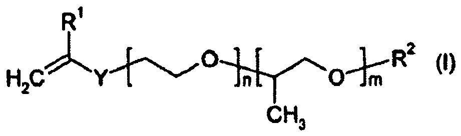 dans laquelle
R¹ signifie hydrogène ou méthyle,
Y signifie O, NR³, CH₂O, CH₂NR³, C(O)O ou C(O)NR³, et de préférence C(O)O ou C(O)NR³,
R² signifie un radical alkyle saturé linéaire ou ramifié contenant 12 à 200, de préférence 12 à 58, de manière particulièrement préférée 12 à 40 et de manière notamment préférée 12 à 30 atomes C ou un radical alcényle mono- ou polyinsaturé linéaire ou ramifié contenant 12 à 200, de préférence 12 à 58, de manière particulièrement préférée 12 à 40 et de manière notamment préférée 12 à 30 atomes C,
R³ signifie hydrogène ou méthyle, et
n et m signifient chacun indépendamment l'un de l'autre un nombre entier de 0 à 200, de préférence de 0 à 100, de manière particulièrement préférée de 0 à 50 et de manière notamment préférée de 0 à 30.

3. Copolymère selon la revendication 1 ou 2, **caractérisé en ce qu'**il contient 60,0 à 99,9 % en poids d'une ou de plusieurs unités structurales du composant a), qui proviennent d'un ou de plusieurs composés polymérisables choisis parmi les formules structurales (Ia) et (Ib) : 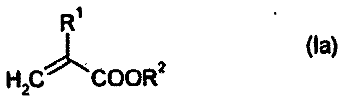 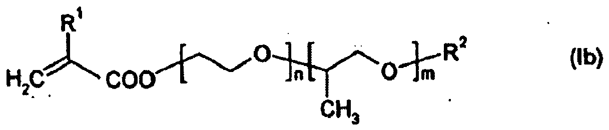 dans lesquelles
R¹ signifie hydrogène ou méthyle,
R² signifie un radical alkyle saturé linéaire ou ramifié contenant 12 à 200, de préférence 12 à 58, de manière particulièrement préférée 12 à 40 et de manière notamment préférée 12 à 30 atomes C ou un radical alcényle mono- ou polyinsaturé linéaire ou ramifié contenant 12 à 200, de préférence 12 à 58, de manière particulièrement préférée 12 à 40 et de manière notamment préférée 12 à 30 atomes C, et
n et m signifient chacun indépendamment l'un de l'autre un nombre entier de 0 à 200, de préférence de 0 à 100, de manière particulièrement préférée de 0 à 50 et de manière notamment préférée de 0 à 30, et la somme n + m est un nombre de 1 à 200, de préférence de 2 à 100, de manière particulièrement préférée de 3 à 50 et de manière notamment préférée de 3 à 30.

4. Copolymère selon la revendication 3, **caractérisé en ce que** les composés de formule (Ia) sont choisis parmi l'acrylate de lauryle, le méthacrylate de lauryle, l'acrylate d'hexadécyle, le méthacrylate d'hexadécyle, l'acrylate de stéaryle, le méthacrylate de stéaryle, l'acrylate d'isostéaryle, le méthacrylate d'isostéaryle, l'acrylate d'octyldodécyle, le méthacrylate d'octyldodécyle, l'acrylate de béhényle et le méthacrylate de béhényle, et les composés de formule (Ib) sont choisis parmi l'ester d'acide acrylique d'un alcool laurylique contenant 7 unités EO, l'ester d'acide méthacrylique d'un alcool laurylique contenant 7 unités EO, l'ester d'acide acrylique d'un alcool talylique contenant 8 unités EO, l'ester d'acide méthacrylique d'un alcool talylique contenant 8 unités EO, l'ester d'acide acrylique d'un alcool talylique contenant 25 unités EO, l'ester d'acide méthacrylique d'un alcool talylique contenant 25 unités EO, l'ester d'acide acrylique d'un alcool béhénylique contenant 25 unités EO et l'ester d'acide méthacrylique d'un alcool béhénylique contenant 25 unités EO.

5. Copolymère selon une ou plusieurs des revendications 1 à 4, **caractérisé en ce que** R² dans les composés de la formule (I) ou dans les composés des formules (Ia) et (Ib) signifie un radical alkyle saturé linéaire ou ramifié, de préférence linéaire, contenant 12 à 200, de préférence 12 à 58, de manière particulièrement préférée 12 à 40 et de manière notamment préférée 12 à 30 atomes C.

6. Copolymère selon une ou plusieurs des revendications 1 à 5, **caractérisé en ce que** les unités structurales du composant b) proviennent de substances choisies parmi :
b1) les composés d'ammonium quaternaires polymérisables de la formule structurale (II) suivante :
X⁻⁺NR⁷R⁸R⁹R¹⁰ (II)
dans laquelle
X⁻ signifie un contre-ion chargé négativement,
R⁷ signifie un radical vinyle, allyle ou de la formule structurale (III) suivante : 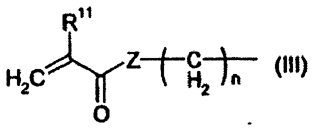 dans laquelle
R¹¹ signifie hydrogène ou méthyle,
Z signifie O, NH, NCH₃ ou S,
n signifie un nombre entier de 1 à 50,
R⁸ signifie un radical alkyle linéaire ou ramifié contenant 1 à 50 atomes C ou un radical R⁷,
R⁹ signifie un radical alkyle linéaire ou ramifié contenant 1 à 50 atomes C ou un radical R⁷, et
R¹⁰ signifie un radical alkyle linéaire ou ramifié contenant 1 à 50 atomes C,
et
b2) les composés d'ammonium quaternaires cycliques polymérisables de la formule structurale (IV) suivante : 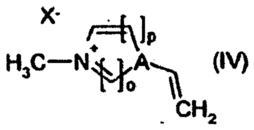
dans laquelle
o et p signifient chacun indépendamment l'un de l'autre un nombre entier de 0 à 5,
A signifie C, CH, N ou P, et
X⁻ signifie un contre-ion chargé négativement.

7. Copolymère selon la revendication 6, **caractérisé en ce que** les contre-ions chargés négativement X⁻ dans les formules (II) et (IV) sont choisis parmi chlorure, bromure, iodure, ½ SO₄²⁻ et méthosulfate.

8. Copolymère selon une ou plusieurs des revendications 1 à 7, **caractérisé en ce que** les unités structurales du composant b) proviennent de composés d'ammonium quaternaires polymérisables choisis parmi le chlorure de diallyldiméthylammonium (DADMAC), le chlorure de [2-(méthacryloyloxy)éthyl]-triméthylammonium (MAOETAC), le chlorure de [2-(acryloyloxy)éthyl]-triméthylammonium (AOETAC), le chlorure de [2-méthacrylamidoéthyl]triméthylammonium, le chlorure de [2-(acrylamido)éthyl]triméthylammonium, le chlorure de [3-méthacryl-amidopropyl]triméthylammonium (MAPTAC), le chlorure de [3-acrylamidopropyl]triméthylammonium (APTAC), le chlorure de N-méthyl-2-vinylpyridinium, le chlorure de N-méthyl-4-vinylpyridinium et le chlorure de N-méthyl-3-vinylimidazolinium.

9. Copolymère selon une ou plusieurs des revendications 6 à 8, **caractérisé en ce que** les unités structurales du composant b) proviennent de composés d'ammonium quaternaires polymérisables de formule structurale (II).

10. Copolymère selon une ou plusieurs des revendications 1 à 9, **caractérisé en ce qu'**il contient une ou plusieurs unités structurales du composant c), qui proviennent de substances choisies parmi les monomères contenant plus d'un groupe polymérisable qui peuvent avoir un effet réticulant.

11. Copolymère selon une ou plusieurs des revendications 1 à 10, **caractérisé en ce que** les unités structurales du composant c) proviennent de substances choisies parmi le divinylbenzène, le méthylènebisacrylamide (MBA), la triallylamine, le cyanurate de triallyle, le diacrylate d'éthanediol, le diméthacrylate d'éthanediol (EDDMA), le diacrylate de butanediol, le diméthacrylate de butanediol (BDDMA), le diacrylate d'hexanediol, le diméthacrylate d'hexanediol (HDDMA), le diacrylate de dodécanediol, le diméthacrylate de dodécanediol (DDDMA), le triacrylate de triméthylolpropane (TMPTA), le triméthacrylate de triméthylolpropane (TMPTMA), le diacrylate de glycérine, le diméthacrylate de glycérine, le diacrylate de tétraéthylène glycol, le diméthacrylate de tétraéthylène glycol, le diacrylate de polyéthylène glycol de masses molaires comprises entre 400 et 800 g/mol, le diméthacrylate de polyéthylène glycol de masses molaires comprises entre 400 et 800 g/mol, l'ester allylique de l'acide acrylique, méthacrylique ou éthacrylique, de préférence l'ester allylique de l'acide acrylique ou méthacrylique et de manière particulièrement préférée l'ester allylique de l'acide méthacrylique (AMA), l'éther diallylique de dipropylène glycol, l'éther diallylique de polyglycol, l'éther diallylique d'hydroquinone, l'éther diallylique de triméthylolpropane, l'éther triallylique de triméthylolpropane, le tétraallyloxyéthane, l'éther divinylique de triéthylène glycol et d'autres éthers allyliques ou vinyliques d'alcools multifonctionnels, ainsi que les mélanges des substances susmentionnées.

12. Copolymère selon une ou plusieurs des revendications 1 à 11, **caractérisé en ce que** la quantité d'unités structurales du composant a) est de 60,0 à 99,5 % en poids, la quantité d'unités structurales du composant b) est de 0,5 à 20,0 % en poids et la quantité d'unités structurales du composant c) est de 0 à 20,0 % % en poids.

13. Copolymère selon une ou plusieurs des revendications 1 à 12, **caractérisé en ce que** la quantité d'unités structurales du composant a) est de 80,0 à 98,0 % en poids, la quantité d'unités structurales du composant b) est de 0,7 à 11,0 % en poids et la quantité d'unités structurales du composant c) est de 0 à 11,0 % en poids.

14. Copolymère selon une ou plusieurs des revendications 3 à 5, **caractérisé en ce que** le rapport en poids unités structurales provenant de composés de formule (Ia):unités structurales provenant de composés de formule (Ib) dans les copolymères est 1,0:0 à 0,8.

15. Copolymère selon une ou plusieurs des revendications 1 à 14, **caractérisé en ce qu'**il ne contient pas d'unité structurale provenant de substances polymérisables anioniques.

16. Copolymère selon une ou plusieurs des revendications 1 à 15, **caractérisé en ce qu'**il est constitué par les unités structurales mentionnées selon les composants a) et b).

17. Copolymère selon une ou plusieurs des revendications 1 à 15, **caractérisé en ce qu'**il est constitué par les unités structurales mentionnées selon les composants a), b) et c).

18. Copolymère selon la revendication 17, **caractérisé en ce que** les unités structurales du composant c) sont choisies parmi des unités structurales réticulantes.

19. Procédé de fabrication d'un copolymère selon une ou plusieurs des revendications 1 à 18, **caractérisé en ce que** les monomères sont polymérisés par voie radicalaire.

20. Formulation cosmétique, pharmaceutique ou dermatologique contenant un ou plusieurs copolymères selon une ou plusieurs des revendications 1 à 18.

21. Utilisation d'un ou de plusieurs des copolymères selon une ou plusieurs des revendications 1 à 18 pour l'épaississement d'une huile.

22. Utilisation d'un ou de plusieurs des copolymères selon une ou plusieurs des revendications 1 à 18 pour le conditionnement de fibres, de préférence de fibres kératiniques.

23. Utilisation d'un ou de plusieurs des copolymères selon une ou plusieurs des revendications 1 à 18 pour augmenter la résistance à l'eau de formulations de protection solaire.

24. Utilisation d'un ou de plusieurs des copolymères selon une ou plusieurs des revendications 1 à 18 pour augmenter le facteur de protection solaire de formulations de protection solaire.

25. Utilisation d'un ou de plusieurs des copolymères selon une ou plusieurs des revendications 1 à 18 en tant qu'émulsifiant.
